# EUROPEAN PATENT SPECIFICATION

(11) **EP 0 372 268 B1**
(45) Date of publication and mention of the grant of the patent: **04.05.1994**
(21) Application number: 89121139.3
(22) Date of filing: 15.11.1989
(51) Int. Cl.: C07H 19/04, C07H 19/173, A61K 31/70, C07H 19/073

(54) **Novel 2'-halomethylidene, 2'-ethenylidene and 2'-ethynyl cytidine, uridine and guanosine derivatives**
Derivate von 2'-Halomethyliden, 2'-Ethenyliden und 2'-Ethynylcytidin, Uridin und Guanosin
Dérivés de 2'-halométhylidène et 2'-éthénylidène et 2'-éthynyl cytidine, uridine et guanosine

(30) Priority: 15.11.1988 US 271479
(43) Date of publication of application: 13.06.1990
(73) Proprietor: MERRELL DOW PHARMACEUTICALS INC., Cincinnati Ohio 45215-6300 (US)
(72) Inventor: McCarthy, James R., West Chester Ohio 45069 (US); Edwards, Michael L., Cincinnati Ohio 45240 (US); Matthews, Donald P., West Chester Ohio 45069 (US)
(74) Representative: Sgarbi, Renato

(56) References cited:
- EP-A- 0 310 673
- WO-A-88/07049

## Description

### SUMMARY OF THE INVENTION

The present invention relates to novel 2′-halomethylidene, 2′-ethenylidene and 2′-ethynyl cytidine, uridine and guanosine derivatives which are useful as anti-viral and anti-neoplastic agents.

The present invention provides novel 2′-halomethylidene derivatives of the formula (1) 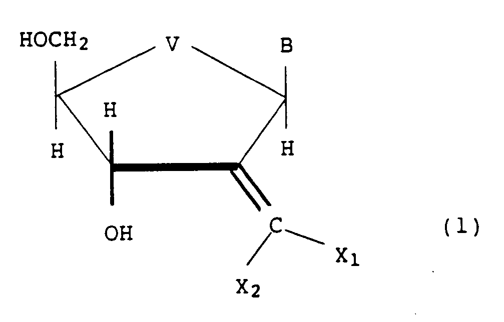
wherein
V is oxy, methylene, or thio,
X₁ and X₂ are each independently hydrogen or halogen,
with the proviso that at least one of X₁ and X₂ is halogen,
B is a radical of the formula 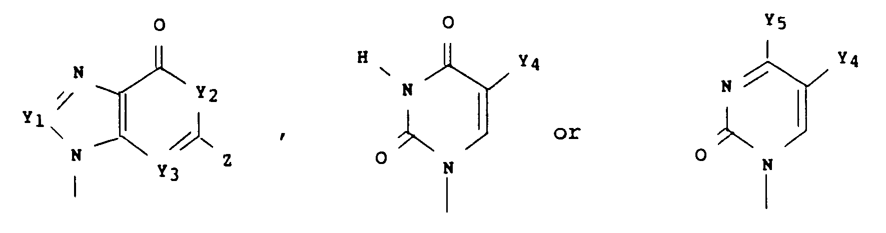
wherein Y₁ is nitrogen, a CH group, a CCl group, a CBr group or a CNH₂ group; Y₂ and Y₃ are each independently nitrogen or a CH group; Y₄ is hydrogen, C₁-C₄ alkyl, C₁-C₄ alkoxy or halogen; Y₅ is amino or C₁-C₄ alkoxy; and Z is hydrogen, halogen, or NH₂;
or a pharmaceutically acceptable salt thereof.

In addition, the present invention also provides novel 2′-ethenylidene derivatives of the formula (1a) 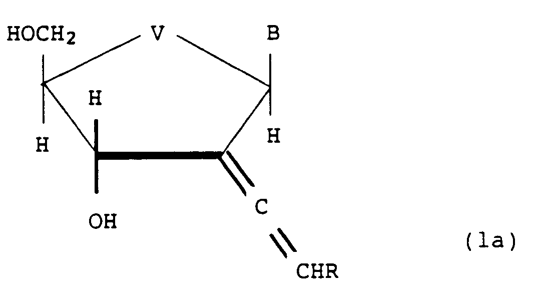
wherein
R is hydrogen or C₁-C₄ alkyl,
and V and B are as previously defined;
or a pharmaceutically acceptable salt thereof.

Furthermore, the present invention provides novel 2′-ethynyl derivatives of the formula (1b) 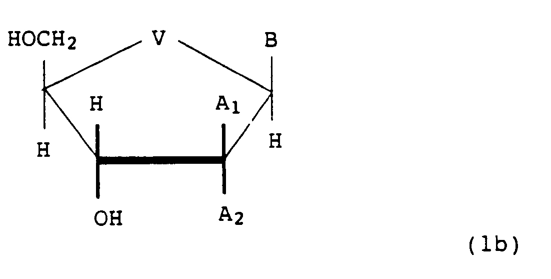
wherein
A₁ and A₂ are each independently hydrogen or a -C≡CR group, with the proviso that where A₁ is hydrogen A₂ is a -C≡CR group, and where A₁ is a -C≡CR group A₂ is hydrogen, and V, R and B are as previously defined;
or a pharmaceutically acceptable salt thereof.

The present invention also provides novel 2′-arylsulfonylmethylidene derivatives of the formula (6) 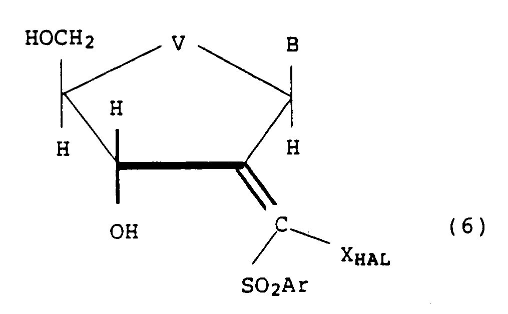
wherein Ar is a C₆-C₁₂ aryl group, X_{HAL} is halogen, and V and B are as previously defined. The compounds of formula (6) are useful as chemical intermediates in the synthesis of compounds of formula (1) wherein one of X₁ or X₂ is hydrogen.

Another embodiment of the present invention is a method of treating a patient afflicted with a neoplastic disease state or of controlling the growth of a neoplasm in a patient afflicted with a neoplastic disease state comprising administration of a therapeutically effective antineoplastic dose of a compound of formula (1), (1a) or (1b).

A further embodiment of the present invention is a method of treating a patient afflicted with a viral infection or of controlling a viral infection in a patient afflicted therewith comprising administration of a therapeutically effective antiviral amount of a compound of formula (1), (1a) or (1b).

### DETAILED DESCRIPTION OF THE INVENTION

As used herein, the term "halogen" or "halo-" refers to a fluorine, chlorine, bromine, or iodine atom and the term "nitrogen" refers to a trivalent nitrogen atom attached to two radicals. The term "C₁-C₄ alkyl" refers to a saturated straight or branched chain hydrocarbyl radical of one to four carbon atoms and includes methyl, ethyl, propyl, isopropyl, n-butyl, isobutyl, tertiary butyl and the like.

The term "C₆-C₁₂ aryl group" refers to an aromatic hydrocarbon of from about 6 to about 12 carbon atoms such as phenyl, naphthyl or phenyl(C₁-C₄)alkyl groups wherein said groups are optionally substituted with one, two or three substituents selected from the group consisting of C₁-C₄ alkyl, halo-substituted C₁-C₄ alkyl, halogen or C₁-C₄ alkoxy. The term "phenyl(C₁-C₄)alkyl" refers to a phenyl group substituted with a C₁-C₄ alkyl including phenylmethyl and phenethyl groups. The term "halo-substituted C₁-C₄ alkyl" refers to a C₁-C₄ alkyl group substituted with one to three halogen atoms including fluorine and chlorine. The term "C₁-C₄ alkoxy" refers to a C₁-C₄ alkyl bearing an oxy group and includes methoxy, ethoxy, propoxy, butoxy and the like.

The 2′-halomethylidene, 2′-ethenylidene and 2′-ethynyl derivatives of formula (1), (1a) or (1b) can be prepared by utilizing procedures and techniques well known and appreciated by one of ordinary skill in the art.

A general synthetic procedure for the preparation of compounds of formula (1) wherein both X₁ and X₂ are halogen is set forth in Scheme A. In the following schemes all substituents, unless otherwise indicated, are as previously defined. In addition, the term "φ" refers to a phenyl group; the term "X_{HAL}" refers to a halogen atom; the term "LP=" indicates a phosphorus ylide moiety [for example, a difluoromethylidene phosphonate ylide can have the formula (φ)₂P(O)=C(F)₂ which can thus be abbreviated as LP=C(F)₂]. 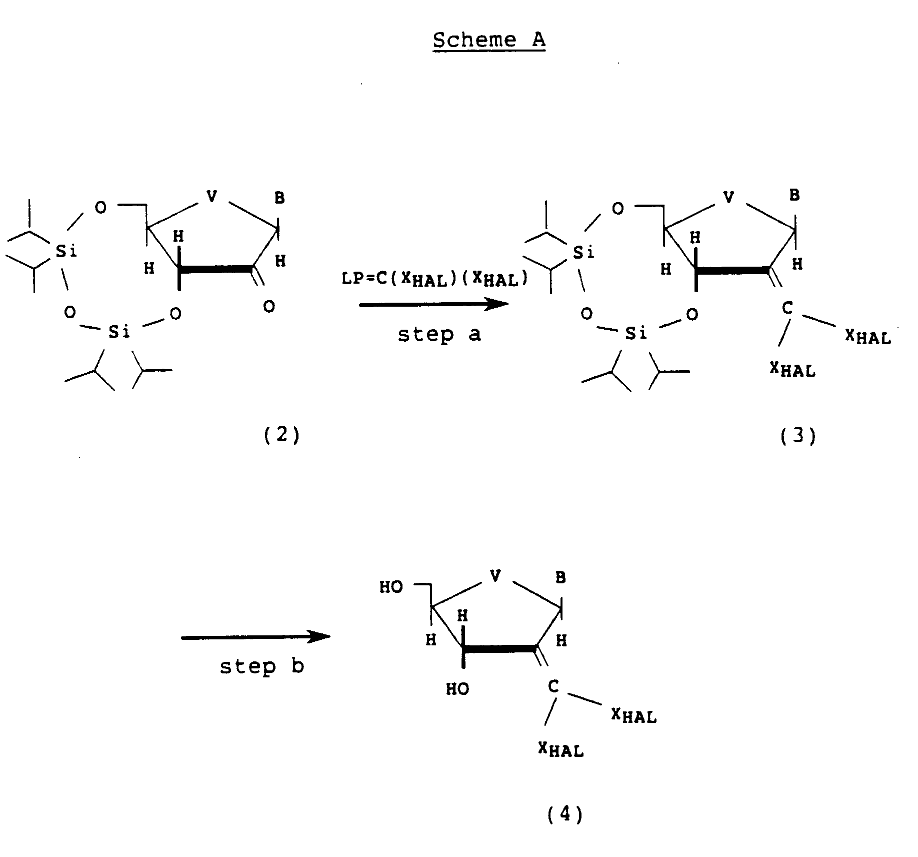
In step a, the ketone derivative (2), for example, 4-ethoxy-1-[(3,5-O-tetraisopropyldisiloxan-1,3-diyl)-β-D-erythro-pentofuran-2-keto-osyl]-2(1H)-pyrimidone, can be reacted in a Wittig type reaction with a dihalomethylidene phosphorus ylide, to yield the corresponding 2-dihalomethylidene substituted derivative (3), for example, 4-ethoxy-1-[(3,5-O-tetraisopropyldisiloxan-1,3-diyl)-β-D-erythro-pentofuran-2-difluoromethylidene-osyl]-2(1H)-pyrimidone.

Phosphorus ylides can be prepared according to procedures which are well known and appreciated in the art of chemistry such as those described by J. March in "Advanced Organic Chemistry: Reactions, Mechanisms and Structure", McGraw-Hill Book Company, 702-10 (1968). For example, a phosphorus ylide can be prepared by treatment of an appropriate phosphorane or phosphonate derivative with an appropriate base. A wide variety of bases can be used including alkoxides and organometallics, such as alkyllithium or lithium dialkylamide. When a compound of formula (1) is desired wherein both X₁ and X₂ are halogen, a dihalomethylidene phosphorus ylide is utilized in step a.

Appropriate phosphoranes or phosphonates can be prepared by addition of phosphines or phosphine oxides, such as trialkylphosphine, triarylphosphine (including triphenylphosphine) and diarylphosphine oxide (including diphenylphosphine oxide), to the appropriate di- or trihalomethane derivative. The appropriate phosphorane or phosphonate is converted to the corresponding phosphorus ylide by treating the phosphorane or phosphonate with base. This can be accomplished by carrying out the preparation of the phosphorane or phosphonate in the presence of an appropriate base. When a compound of formula (1) is desired wherein both X₁ and X₂ are halogen, the appropriate ketone (2) can be reacted with a dihalomethylidene phosphorus ylide, prepared by reacting a phosphine or phosphine oxide with a trihalomethane in the presence of base.

More specifically, when a compound of formula (1) is desired wherein both X₁ and X₂ are fluorine, the appropriate ketone (2) is reacted with a difluoromethylidene phosphorus ylide, prepared by reacting a phosphine or phosphine oxide (such as diphenylphosphine oxide) with a difluorohalomethane (such as difluorochloromethane) in the presence of base (such as butyllithium).

In step b, the tetraisopropyldisiloxan blocking group of (3) is removed, according to conventional procedures and techniques well known and appreciated in the art, to yield the de-blocked dihalomethylidene derivative (4). The tetraisopropyldisiloxan blocking group can be removed by reacting (3) with a fluoride anion or acid to effectively remove the blocking group without degradation of the desired product. For example, tetrabutylammonium fluoride, dilute acetic acid or dilute hydrochloric acid can be used.

Where a 4-alkoxy-substituted pyrimidone, such as the 4-ethoxy-substituted pyrimidone, is utilized as the starting material (2) for step a, the 4-alkoxy moiety of the pyrimidone base can be converted, in step b, to the 4-keto moiety to yield the corresponding uridine or thymidine derivative (4) or it can be converted to the 4-amino moiety to yield the corresponding cytidine derivative (4). These reactions can be effected according to procedures well known and appreciated in the art of chemistry. For example, where a uridine or thymidine derivative is desired, the 4-ethoxy-dihalomethylidine derivative of (4) can be treated with a base, such as sodium hydroxide, to convert the 4-ethoxy moiety to a 4-keto moiety in a nucleophilic displacement/enolization reaction. Where a cytidine derivative is desired, the 4-ethoxy-dihalomethylidine derivative of (4) can be reacted with methanolic ammonia to effect the substitution of an NH₂ group for the 4-ethoxy moiety.

The following example presents a typical synthesis as described by Scheme A. This example is understood to be illustrative only and is not intended to limit the scope of the present invention in any way.

### EXAMPLE 1

### 2′-DEOXY-2′-DIFLUOROMETHYLIDENECYTIDINE

### Step a: 4-Ethoxyl-1-[(3,5-O-tetraisopropyldisiloxan-1,3-diyl)-β-D-erythro-pentofuran-2-(difluoromethylidene)osyl]-2(1H)-pyrimidone

Prepare diphenyldifluoromethylphosphine oxide as follows: To a solution of diphenylphosphine oxide [25 grams (gm), 124 millimoles (mmol)] in tetrahydrofuran (THF) [600 milliliters (ml)] which has been cooled to -50 °Celsius (°C), add 70ml of a solution of 1.8 molar (M) n-butyl lithium in hexane and allow to stand at -50°C for 20 minutes (min). Add an excess of difluorochloromethane slowly and stir at -50°C for 3 hours. Allow the mixture to come to room temperature and evaporate the solvent *in vacuo*. Redissolve the residue in chloroform/water (1/1, v/v; 200ml). Separate the organic layer, dry with anhydrous magnesium sulfate, and evaporate to dryness. Purify by flash chromatography on silica gel eluting with toluene/ethyl acetate (1/1, v/v). Recrystallize from hexane/dichloromethane to yield the purified diphenyldifluoromethylphosphine oxide (melting point 93-94°C).

Cool diisopropylamine [1.7 ml, 12 mmol] in THF (24 ml) to -20°C in a chloroform/dry ice bath. Add n-butyl lithium [8.88 ml of a 1.35 molar (M) solution in hexane] in a dropwise manner and stir the mixture for 20 min. Cool the mixture to - 70°C in an acetone/dry ice bath. Add diphenyldifluoromethylphosphine oxide [3.02 gm, 12 mmol] in THF (12 ml) in a dropwise manner at such a rate that the temperature of the mixture does not rise above -65°C. Stir the mixture for 30 min and then add 4-ethoxy-1-[(3,5-O-tetraisopropyldisiloxan-1,3-diyl)-2-keto-β-D-erythro-pentofuranosyl]-2(1H)-pyrimidone (5.12 gm, 10 mmol) in THF (20 ml) in a dropwise manner. Stir the mixture for 1 hour at -70°C, gradually warm the mixture to room temperature and then reflux for 1/2 hour. Cool the mixture to room temperature, add ethyl acetate (500 ml) and wash the organic mixture with saturated aqueous sodium bicarbonate (100 ml). Separate the organic layer, dry with anhydrous magnesium sulfate, and evaporate to dryness *in vacuo*. Chromatograph the residue on a silica gel flash column eluting with ethyl acetate/hexane (1/1, v/v) to yield the title compound.

### Step b: 2′-Deoxy-2′-difluoromethylidenecytidine

To a solution of 1.0 M tetrabutylammonium fluoride in THF (2.2 ml, 2.2 mmol) add 4-ethoxy-1-[(3,5-O-tetraisopropyldisiloxan-1,3-diyl)-β-D-erythro-pentofuran-2-(difluoromethylidene)osyl]-2(1H)-pyrimidone (546 mg, 1 mmol) and stir the mixture at room temperature for 2 hours. Neutralize the mixture with acetic acid, add flash silica gel to the mixture and evaporate to dryness *in vacuo*. Apply the residue to a flash silica gel column and elute with chloroform/ethanol (9/1, v/v) to provide 4-ethoxy-1-[β-D-erythro-pentofuran-2-(difluoromethylidene)osyl]-2(1H)-pyrimidone.

Heat a solution of 4-ethoxyl-1-[β-D-erythro-pentofuran-2-(difluoromethylidene)osyl]-2(1H)-pyrimidone (909 mg, 3 mmol) in methanolic ammonia (10 ml, saturated at 0°C) in a sealed tube at 100°C for 2 days. Evaporate the solution to dryness to provide the title compound.

The following compounds can be made by procedures analogous to those described above in Example 1:
2′-deoxy-2′-difluoromethylidene-5-methylcytidine
2′-deoxy-2′-difluoromethylidene-5-hydroxymethylcytidine
2′-deoxy-2′-dichloromethylidenecytidine
2′-deoxy-2′-difluoromethylidene-4′-thiocytidine
(±)-(1β,3α,4β)-1-(4-amino-2-hydroxypyrimidin-6-yl)-2-difluoromethylidene-3-hydroxy-4-hydroxymethylcyclopentane

### EXAMPLE 2

### 2′-DEOXY-2′-DIFLUOROMETHYLIDENEURIDINE

### Step a: 4-Ethoxy-1-[(3,5-O-tetraisopropyldisiloxan-1,3-diyl)-β-D-erythro-pentofuran-2-(difluoromethylidene)osyl]-2(1H)-pyrimidone

Prepare the title compound as described in Example 1, step a.

### Step b: 2′-Deoxy-2′-difluoromethylideneuridine

Prepare 4-ethoxy-1-[β-D-erythro-pentofuran-2-(difluoromethylidene)osyl]-2(1H)-pyrimidone as described in Example 1, step b.

Stir a solution of 4-ethoxy-1-[β-D-erythropento-furan-2-(difluoromethylidene)osyl]-2(1H)-pyrimidone (608 mg, 2 mmol) in THF (15 ml) and 1N sodium hydroxide (5 ml) at room temperature for 23 hours and then at 60°C for 2 hours. Neutralize the reaction solution with Amberlite IRC-50 (H⁺-form) and filter off the resin. Evaporate the filtrate to dryness to provide the title compound.

The following compounds can be made by procedures analogous to those described above in Example 2:
2′-deoxy-2′-difluoromethylidenethymidine
2′-deoxy-2′-difluoromethylidene-5-hydroxymethyluridine
2′-deoxy-2′-dichloromethylideneuridine
2′-deoxy-2′-difluoromethylidene-4′-thiouridine
(±)-(1β,3α,4β)-1-(2,4-dihydroxypyrimidin-6-yl)-2-difluoromethylidene-3-hydroxy-4-hydroxymethylcyclopentane

### EXAMPLE 3

### 2′-DEOXY-2′-DIFLUOROMETHYLIDENEGUANOSINE

### Step a: 1-[(3,5-O-tetraisopropyldisiloxan-1,3-diyl)-β-D-erythro-pentofuran-2-(difluoromethylidene)osyl]guanine

Prepare the title compound as described in Example 1, step a, from 1-[(3,5-O-tetraisopropyldisiloxan-1,3-diyl)-2-keto-β-D-erythro-pentofuranosyl]guanine (5.16 gm, 10 mmol).

### Step b: 2′-Deoxy-2′-difluoromethylideneguanosine

Prepare the title compound as described in Example 1, step b, from 9-[(3,5-O-tetraisopropyldisiloxan-1,3-diyl)-β-D-erythro-pentofuran-2-(difluoromethylidene)osyl]guanine (0.55 gm, 1 mmol).

The following compounds can be made by procedures analogous to those described above in Example 3:
2′-deoxy-2′-dichloromethylideneguanosine
2′-deoxy-2′-difluoromethylidene-4′-thioguanosine
A general synthetic procedure for the preparation of compounds of formula (1) wherein one of X₁ and X₂ is hydrogen is set forth in Scheme B. 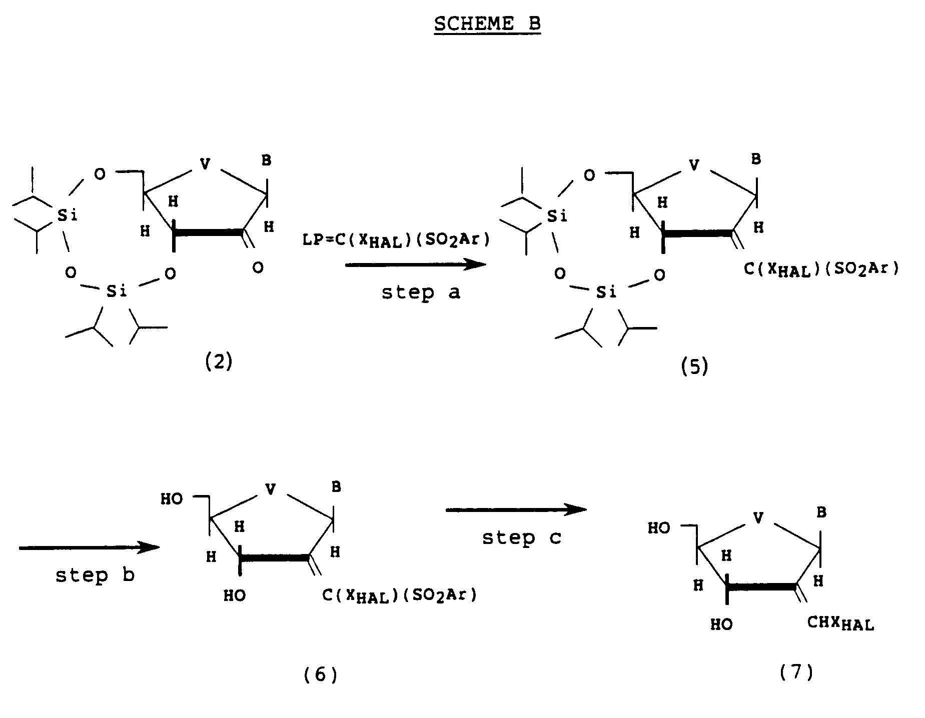
In step a, the ketone derivative (2), for example, 4-ethoxy-1-[(3,5-O-tetraisopropyldisiloxan-1,3-diyl)-β-D-erythro-pentofuran-2-keto-osyl]-2(1H)-pyrimidone, is reacted in a Wittig reaction with a phosphorus ylide as generally described for Scheme A. When a compound of formula (1) is desired wherein one of X₁ and X₂ is hydrogen, (2) can be reacted with an arylsulfonylhalomethylidene phosphorus ylide, to yield the corresponding 2-arylsulfonylhalomethylidene derivative (5) such as the 2-phenylsulfonylhalomethylidene derivative.

The appropriate arylsulfonylhalomethylidene phosphorus ylide can be prepared according to procedures which are well known and appreciated in the art of chemistry. For example, where a compound of formula (1) is desired wherein one of X₁ and X₂ is hydrogen, the appropriate ketone (2) can be reacted with an arylsulfonylhalomethylidene phosphorus ylide prepared by reacting a halo phosphate (such as diethylchloro phosphate) with a halomethylarylsulfone in the presence of a base (such as lithium diisopropylamide).

More specifically, when a compound of formula (1) is desired wherein one of X₁ and X₂ is fluorine and the other is hydrogen, the appropriate ketone (2) can be reacted with an arylsulfonylfluoromethylidene phosphorus ylide prepared by reacting a halo phosphate (such as diethylchloro phosphate) with fluoromethylarylsulfone in the presence of a base (such as lithium diisopropylamide).

In step b, the tetraisopropyldisiloxan blocking group of (5) is removed as described for Scheme A (step b), to yield the corresponding de-blocked 2-arylsulfonylhalomethylidene-substituted derivative (6). For example, a fluoride salt such as tetrabutylammonium fluoride can be used to remove the tetraisopropyldisiloxan blocking group.

In step c, the arylsulfonyl moiety of (6) is removed and replaced by a hydrogen atom to provide the corresponding 2-halomethylidene derivative (7). This can be accomplished according to procedures well known and appreciated in the art of chemistry, such as reacting (6) with an aluminum/mercury amalgam or a sodium/mercury amalgam or tributyltinhydride/azobisisobutylnitrile (AIBN) in refluxing benzene followed by an aqueous acid treatment.

The 2-arylsulfonylhalomethylidene-substituted derivative (6) is a novel compound useful as a chemical intermediate in the synthesis of compounds of formula (1) wherein one of X₁ or X₂ is hydrogen. The designation "Ar" in the compounds of formula (6) refers to a C₆-C₁₂ aryl group as defined hereinabove. The preferred compounds of formula (6) are those wherein Ar is phenyl.

Where a 4-alkoxy-substituted pyrimidone, such as the 4-ethoxy-substituted pyrimidone, is utilized as the starting material (2) for step a, the 4-alkoxy moiety of the pyrimidone base can be converted, in step c, to the 4-keto moiety to yield the corresponding uridine or thymidine derivative (7) or it can be converted to the 4-amino moiety to yield the corresponding cytidine derivative (7). These reactions can be effected according to procedures well known and appreciated in the art of chemistry as described for Scheme A.

As is readily apparent to one skilled in the art, the 2′-halomethylidene derivative represented by the compound (7) in Scheme B, exists as two geometric isomers which can be referred to as the (Z) and the (E) isomers. These isomers can be separated by using conventional separation techniques well known and appreciated in the art. For example, the geometric isomers may be resolved by column chromatography using a Dowex 1-X2 (OH⁻ form) resin.

The following example presents a typical synthesis as described by Scheme B. This example is understood to be illustrative only and is not intended to limit the scope of the present invention in any way.

### EXAMPLE 4

### (Z)- and (E)-2′-DEOXY-2′-FLUOROMETHYLIDENECYTIDINE

### Step a: 4-Ethoxy-1-[(3,5-O-tetraisopropyldisiloxan-1,3-diyl)-β-D-erythro-pentofuran-2-(2-fluoro-2-phenylsulfonyl methylidene)osyl]-2(1H)-pyrimidone

Prepare diethylfluoromethylphenylsulfonylphosphonate as follows: To a solution of fluoromethylphenyl sulfone (500 mg, 2.87 mmol) in dry THF (30 ml) which has been cooled to about - 60°C in a dry 3-necked 100ml flask with stirring bar, argon inlet valve, thermometer and rubber septum, add diethyl chlorophosphate (500 mg, 0.42 ml, 2.87 mmol) via syringe. To this mixture, add a solution of 1.65 M lithium diisopropylamide in cyclohexane (3.48 ml, 5.74 mmol) via syringe and follow the formation of diethylfluoromethylphenylsulfonylphosphonate by gas-liquid chromatography (GLC).

To the diethylfluoromethylphenylsulfonylphosphonate solution above add a solution of 4-ethoxy-1-[(3,5-O-tetraisopropyldisiloxan-1,3-diyl)-2-keto-β-D-erythro-pentofuranosyl]-2(1H)-pyrimidone (732 mg, 2 mmol) in dry THF (about 5 ml) and allow the reaction mixture to warm to room temperature overnight under an argon atmosphere. Pour the mixture into a saturated, ice-cold solution of ammonium chloride and extract the mixture with ethyl acetate (3 times, 75 ml each time). Combine the organic layers, dry with anhydrous magnesium sulfate, and evaporate to dryness. Chromatograph the residue on a silica gel flash column eluting with ethyl acetate/hexane (1/1, v/v) to provide the title compound.

### Step b: 4-Ethoxy-1-[β-D-erythro-pentofuran-2-(2-fluoro-2-phenylsulfonylmethylidene)osyl]-2(1H)-pyrimidone

To a solution of 1.0 M tetrabutylammonium fluoride in THF (2.2 ml, 2.2 mmol) add 4-ethoxy-1-[(3,5-O-tetraisopropyldisiloxan-1,3-diyl)-β-D-erythro-pentofuran-2-(2-fluoro-2-phenylsulfonylmethylidene)osyl]-2(1H)-pyrimidone (668 mg, 1 mmol) and stir the mixture at room temperature for 2 hours. Neutralize the mixture with acetic acid, add flash silica gel to the mixture and evaporate to dryness *in vacuo*. Apply the residue to a flash silica gel column and elute with chloroform/ethanol (20/1, v/v) to provide the title compound.

### Step c: (Z)- and (E)-2′-Deoxy-2′-fluoromethylidenecytidine

To a solution of 4-ethoxy-1-[β-D-erythro-pentofuran-2-(2-fluoro-2-phenylsulfonylmethylidene)osyl]-2(1H)-pyrimidone (854 mg, 2 mmol) in 10% aqueous THF (100 ml) under a nitrogen atmosphere, add aluminum amalgam (made from 0.04 gm aluminum in 2% aqueous HgCl₂). Stir and vent the mixture while reluxing for 2 hours. Filter the mixture and evaporate most of the THF *in vacuo*. Extract the residue with ethyl acetate (3 times, 25 ml each time), combine the organic layers and dry with anhydrous Na₂SO₄. Evaporate to dryness *in vacuo* and apply the residue to a flash silica gel column and elute with chloroform/ethanol (9/1, v/v) to provide (Z)- and (E)-4-ethoxy-1-[β-D-erythro-pentofuran-2-(2-fluoromethylidene)osyl]-2(1H)-pyrimidone as a mixture of geometric isomers.

Heat a solution of (Z)- and (E)-4-ethoxy-1-[β-D-erythro-pentofuran-2-(2-fluoromethylidene)osyl]-2(1H)-pyrimidone (858 mg, 3 mmol) in methanolic ammonia (10 ml, saturated at 0°C) in a sealed tube at 100°C for 2 days. Evaporate the solution to dryness and separate the (Z) and (E) isomers of the title compound by chromatography by applying the residue to a column packed with Dowex 1-X2 (OH⁻ form) and eluting with methanol.

The following compounds can be made by procedures analogous to those described above in Example 4:
(E) and (Z)-2′-deoxy-2′-fluoromethylidene-5-methylcytidine
(E) and (Z)-2′-deoxy-2′-fluoromethylidene-5-hydroxymethylcytidine
(E) and (Z)-2′-deoxy-2′-chloromethylidenecytidine
(E) and (Z)-2′-deoxy-2′-fluoromethylidene-4′-thiocytidine
(±)-(1β,3α,4β)-1-(4-amino-2-hydroxypyrimidin-6-yl)-2(E and Z)-fluoromethylidene-3-hydroxy-4-hydroxymethylcyclopentane

### EXAMPLE 5

### (Z)- and (E)-2′-DEOXY-2′-FLUOROMETHYLIDENEURIDINE

### Step a: 4-Ethoxy-1-[(3,5-O-tetraisopropyldisiloxan-1,3-diyl)-β-D-erythro-pentofuran-2-(2-fluoro-2-phenylsulfonyl methylidene)osyl]-2(1H)-pyrimidone

Prepare the title compound as described in Example 4, step a.

### Step b: 4-Ethoxy-1-[β-D-erythro-pentofuran-2-(2-fluoro-2-phenylsulfonylmethylidene)osyl]-2(1H)-pyrimidone

Prepare the title compound as described in Example 4, step b.

### Step c: (Z)- and (E)-2′-Deoxy-2′-fluoromethylideneuridine

Prepare 4-ethoxy-1-[β-D-erythro-pentofuran-2-(2-fluoromethylidene)osyl]-2(1H)-pyrimidone as described in Example 4, step c.

Stir a solution of 4-ethoxy-1-[β-D-erythro-pentofuran-2-(2-fluoromethylidene)osyl]-2(1H)-pyrimidone (572 mg, 2 mmol) in THF (15 ml) and 1N sodium hydroxide (5 ml) at room temperature for 23 hours and then at 60°C for 2 hours. Neutralize the reaction solution with Amberlite IRC-50 (H⁺-form) and filter off the resin. Evaporate the filtrate to dryness to provide the title compounds. Separate the (Z) and (E) isomers of the title compound by chromatography by applying the residue to a column packed with Dowex 1-X2 (OH⁻ form) and eluting with 0.1 M ammonium bicarbonate.

The following compounds can be made by procedures analogous to those described above in Example 5:
(E) and (Z)-2′-deoxy-2′-fluoromethylidene-5-hydroxymethyl uridine
(E) and (Z)-2′-deoxy-2′-fluoromethylidenethymidine
(E) and (Z)-2′-deoxy-2′-chloromethylideneuridine
(E) and (Z)-2′-deoxy-2′-fluoromethylidene-4′-thiouridine

### EXAMPLE 6

### (Z)- and (E)-2′-DEOXY-2′-FLUOROMETHYLIDENEGUANOSINE

### Step a: 9-[(3,5-O-tetraisopropyldisiloxan-1,3-diyl)-β-D-erythro-pentofuran-2-(2-fluoro-2-phenylsulfonyl methylidene)osyl]guanine

Prepare the title compound from 9-[(3,5-O-tetraisopropyldisiloxan-1,3-diyl)-2-keto-β-D-erythro-pentofuranosyl]guanine (5.16 gm, 10 mmol) as described in Example 4, step a, .

### Step b: 9-[β-D-erythro-pentofuran-2-(2-fluoro-2-phenylsulfonylmethylidene)osyl]guanine

Prepare the title compound from 9-[(3,5-O-tetraisopropyldisiloxan-1,3-diyl)-β-D-erythro-pentofuran-2-(2-fluoro-2-phenylsulfonylmethylidene)osyl]guanine as described in Example 4, step b.

### Step c: (Z)- and (E)-2′-Deoxy-2′-fluoromethylidenequanosine

Prepare the title compound from 9-[β-D-erythro-pentofuran-2-(2-fluoro-2-phenylsulfonylmethylidene)osyl]guanine as described in Example 4, step c.

The following compounds can be made by procedures analogous to those described above in Example 6:
(Z)- and (E)-2′-deoxy-2′-chloromethylideneguanosine
(Z)- and (E)-2′-deoxy-2′-fluoromethylidene-4′-thioguanosine
(±)-(1β,3α,4β)-1-(2-amino-6-hydroxy-9H-purin-9-yl)-2(E and Z)-fluoromethylidene-3-hydroxy-4-hydroxymethylcyclopentane
A general synthetic procedure for the preparation of compounds of formula (1a) is set forth in Scheme C. This scheme is especially useful for compounds of formula (1a) wherein R is hydrogen. 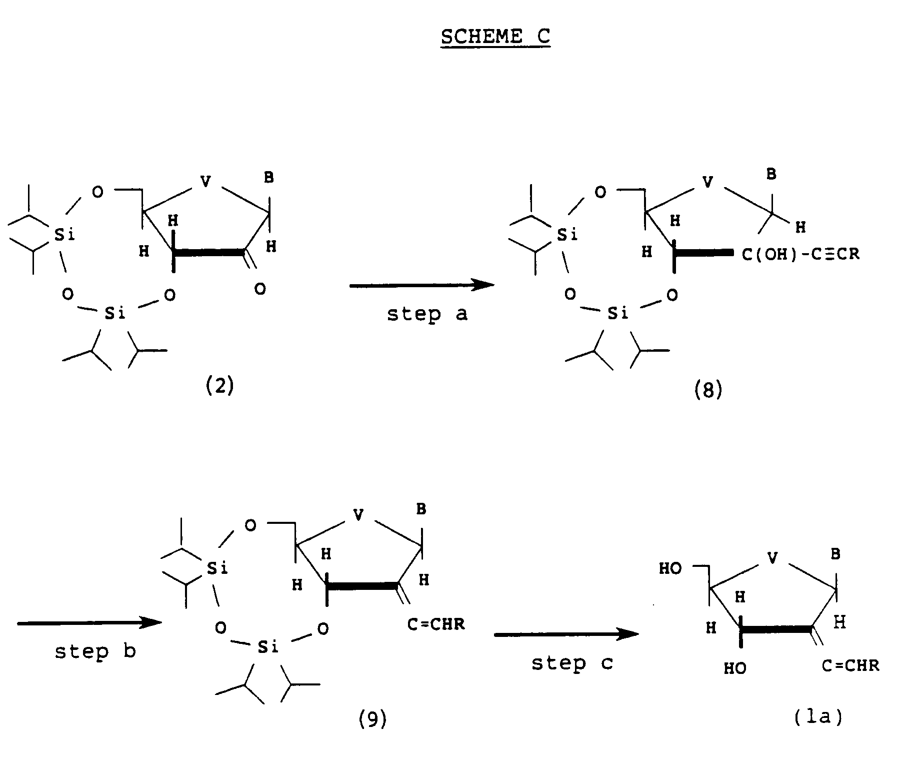
In step a, the ketone derivative (2), for example, 4-ethoxy-1-[(3,5-O-tetraisopropyldisiloxan-1,3-diyl)-β-D-erythro-pentofuran-2-keto-osyl]-2(1H)-pyrimidone, can be reacted with an acetylenic Grignard reagent such as that represented by the general formula RC≡CMgBr, to yield the corresponding 2-ethynyl alcohol (8). Alternatively, the alcohol (8) can be prepared from other organometallic compounds made from reactive metals, such as that represented by the general formula RC≡CLi.

The appropriate Grignard reagent, or other organometallic reagent, can be prepared according to methods and procedures which are well known and appreciated in the art of chemistry such as those described by J. March in "Advanced Organic Chemistry: Reactions, Mechanisms and Structure", McGraw-Hill Book Company, 684-88 and 697-98 (1968). For example, a Grignard reagent of acetylene or an alkyl-substituted acetylene can be prepared by treating acetylene or an alkyl-substituted acetylene with methylmagnesium bromide under anhydrous conditions.

It is of course well appreciated by one skilled in the art that the 2-ethynyl alcohol (8) can exist as one of two diasteriomeric isomers, i.e., one wherein the ethynyl group is on the same side of the furanosyl ring as the 3-hydroxy group and one wherein the ethynyl group is on the same side of the furanosyl ring as the purine or pyrimidine group. Where 4-ethoxy-1-[(3,5-O-tetraisopropyldisiloxan-1,3-diyl)-β-D-erythro-pentofuran2-keto-osyl]-2(1H)-pyrimidone is utilized as the starting material (2), these diasteriomeric isomers are named 4-ethoxy-1-[(3,5-O-tetraisopropyldisiloxan-1,3-diyl)-β-D-ribo-pentofuran2-(ethynyl)osyl]-2(1H)-pyrimidone and 4-ethoxy-1-[(3,5-O-tetraisopropyldisiloxan-1,3-diyl)-β-D-arabino-pentofuran-2-(ethynyl)osyl]-2(1H)-pyrimidone.

In step b, the 2-ethynyl alcohol (8) is reduced to yield the 2-ethenylidene derivative (9). This reduction can be carried out according to methods and procedures which are well known and appreciated in the art of chemistry such as by treating the 2-ethynyl alcohol (8) with lithium aluminum hydride and aluminum chloride.

In step c, the tetraisopropyldisiloxan blocking group of (9) is removed as described for Scheme A (step b), to yield the corresponding de-blocked 2-ethenylidene derivative (1a).

Where a 4-alkoxy-substituted pyrimidone, such as 4-ethoxy-substituted pyrimidone, is utilized as the starting material (2) for step a, the 4-alkoxy moiety of the pyrimidone base can be converted to the 4-keto moiety to yield the corresponding uridine or thymidine derivative (1a) or it can be converted to the 4-amino moiety to yield the corresponding cytidine derivative (1a). These reactions can be effected according to procedures well known and appreciated in the art of chemistry as described for Scheme A.

The following example presents a typical synthesis as described by Scheme C. This example is understood to be illustrative only and is not intended to limit the scope of the present invention in any way.

### EXAMPLE 7

### 2′-DEOXY-2′-ETHENYLIDENECYTIDINE

### Step a: 4-Ethoxy-1-[(3,5-O-tetraisopropyldisiloxan-1,3-diyl)-β-D-(ribo- or arabino-)pentofuran-2-(ethynyl)osyl]-2(1H)-pyrimidone

At 0°C, saturate THF (750 ml) with acetylene and add 1.95N methylmagnesium bromide (51 ml, 0.1 mol) in a dropwise manner while acetylene is still bubbling through the solution. Stop the acetylene stream 20 min after the addition of the methyl magnesium bromide is complete and purge for 20 min with argon. To this solution add 4-ethoxy-1-[(3,5-O-tetraisopropyldisiloxan-1,3-diyl)-β-D-erythro-pentofuran-2-keto-osyl]-2(1H)-pyrimidone (2.73 gm, 5 mmol) in THF (20 ml), warm the reaction mixture to room temperature and stir for 16 hours. Add 1600 ml of ethyl acetate and wash the mixture with saturated aqueous NH₄Cl (200 ml). Dry the organic layer with anhydrous magnesium sulfate and evaporate to dryness *in vacuo*. Chromatograph the residue on a silica gel flash column eluting with ethyl acetate/hexane (1/1, v/v) to provide the title compound.

### Step b: 4-Ethoxy-1-[(3,5-O-tetraisopropyldisiloxan-1,3-diyl)-β-D-erythro-pentofuran-2-(ethenylidene)osyl]-2(1H)-pyrimidone

To a stirred solution of lithium aluminum hydride (76 mg, 2 mmol) and aluminum chloride (132 mg, 1 mmol) in anhydrous diethyl ether (4 ml) which has been cooled to 0°C, add a solution of 4-ethoxy-1-[(3,5-O-tetraisopropyldisiloxan-1,3-diyl)-β-D-(ribo- or arabino-)pentofuran-2-(ethynyl)osyl]-2(1H)-pyrimidone (531 mg, 1 mmol) in anhydrous diethyl ether (2 ml) in a dropwise manner. Stir the reaction mixture for 1 hour and then quench the reaction by adding 10% potassium hydrogen sulfate (10 ml). Wash the aqueous solution with ethyl acetate (3 times, 20 ml each time). Combine the organic layers, dry with anhydrous magnesium sulfate, and concentrate the solution *in vacuo*. Chromatograph the residue on a silica gel flash column eluting with ethyl acetate/hexane (1/1, v/v) to provide the title compound.

### Step c: 2-Deoxy-2′-ethenylidenecytidine

To a solution of 1.0 M tetrabutylammonium fluoride in THF (2.2 ml, 2.2 mmol) add 4-ethoxy-1-[(3,5-O-tetraisopropyldisiloxan-1,3-diyl)-β-D-erythro-pentofuran-2-(ethenylidene)osyl]-2(1H)-pyrimidone (513 mg, 1 mmol) and stir the mixture at room temperature for 2 hours. Neutralize the mixture with acetic acid, add flash silica gel to the mixture and evaporate to dryness *in vacuo*. Apply the residue to a flash silica gel column and elute with chloroform/ethanol (20/1, v/v) to provide 4-ethoxy-1-[β-D-erythro-pentofuran-2-(ethenylidene)osyl]-2(1H)-pyrimidone.

Heat a solution of 4-ethoxy-1-[β-D-erythro-pentofuran-2-(ethenylidene)osyl]-2(1H)-pyrimidone (840 mg, 3 mmol) in methanolic ammonia (10 ml, saturated at 0°C) in a sealed tube at 100°C for 2 days. Evaporate the solution to dryness to provide the title compound.

The following compounds can be made by procedures analogous to those described above in Example 3:
2′-deoxy-2′-ethenylidene-5-methylcytidine
2′-deoxy-2′-ethenylidenes-hydroxymethylcytidine
2′-deoxy-2′-ethenylidene-4′-thiocytidine
(±)-(1β,3α,4β)-1-(4-amino-2-hydroxypyrimidin-6-yl)-2-ethenylidene-3-hydroxy-4-hydroxymethylcyclopentane

### EXAMPLE 8

### 2′-DEOXY-2′-ETHENYLIDENEURIDINE

### Step a: 4-Ethoxy-1-[(3,5-O-tetraisopropyldisiloxan-1,3-diyl)-β-D-(ribo- or arabino-)pentofuran-2-(ethynyl)osyl]-2(1H)-pyrimidone

Prepare the title compound as described in Example 7, step a.

### Step b: 4-Ethoxy-1-[(3,5-O-tetraisopropyldisiloxan-1,3-diyl)-β-D-erythro-pentofuran-2-(ethenylidene)osyl]-2(1H)-pyrimidone

Prepare the title compound as described in Example 7, step b.

### Step c: 2′-Deoxy-2′-ethenylideneuridine

Prepare 4-ethoxy-1-[β-D-erythro-pentofuran-2-(ethenylidene)osyl]-2(1H)-pyrimidone as described in Example 7, step c.

Stir a solution of 4-ethoxy-1-[β-D-erythro-pentofuran-2-(ethenylidene)osyl]-2(1H)-pyrimidone (560 mg, 2 mmol) in THF (15 ml) and 1N sodium hydroxide (5 ml) at room temperature for 23 hours and then at 60°C for 2 hours. Neutralize the reaction solution with Amberlite IRC-50 (H⁺-form) and filter off the resin. Evaporate the filtrate to dryness to provide the title compound.

The following compounds can be made by procedures analogous to those described above in Example 8:
2′-deoxy-2′-ethenylidene-5-hydroxymethyluridine
2′-deoxy-2′-ethenylidenethymidine
2′-deoxy-2′-ethenylidene-4′-thiouridine

### EXAMPLE 9

### 2′-DEOXY-2′-ETHENYLIDENEGUANOSINE

### Step a: 9-[(3,5-O-tetraisopropyldisiloxan-1,3-diyl)-β-D-(ribo-or arabino-)pentofuran-2-(ethynyl)osyl]guanine

Prepare the title compound from 9-[(3,5-O-tetraisopropyldisiloxan-1,3-diyl)-2-keto-β-D-erythro-pentofuranosyl]guanine (2.58 gm, 5 mmol) as described in Example 7, step a.

### Step b: 9-[(3,5-O-tetraisopropyldisiloxan-1,3-diyl)-β-D-erythro-pentofuran-2-(ethenylidene)osyl]guanine

Prepare the title compound from 9-[(3,5-O-tetraisopropyldisiloxan-1,3-diyl)-β-D-(ribo- or arabino-) pentofuran-2-(ethynyl)osyl]guanine as described in Example 7, step b.

### Step c: 2′-Deoxy-2′-ethenylideneguanosine

Prepare the title compound from 9-[(3,5-O-tetraisopropyldisiloxan-1,3-diyl)-β-D-erythro-pentofuran-2-(ethenylidene)osyl]guanine as described in Example 7, step c.

The following compounds can be made by procedures analogous to those described above in Example 9:
2′-deoxy-2′-ethenylidene-4′-thioguanosine

A general synthetic procedure for the preparation of compounds of formula (1b) is set forth in Scheme D. 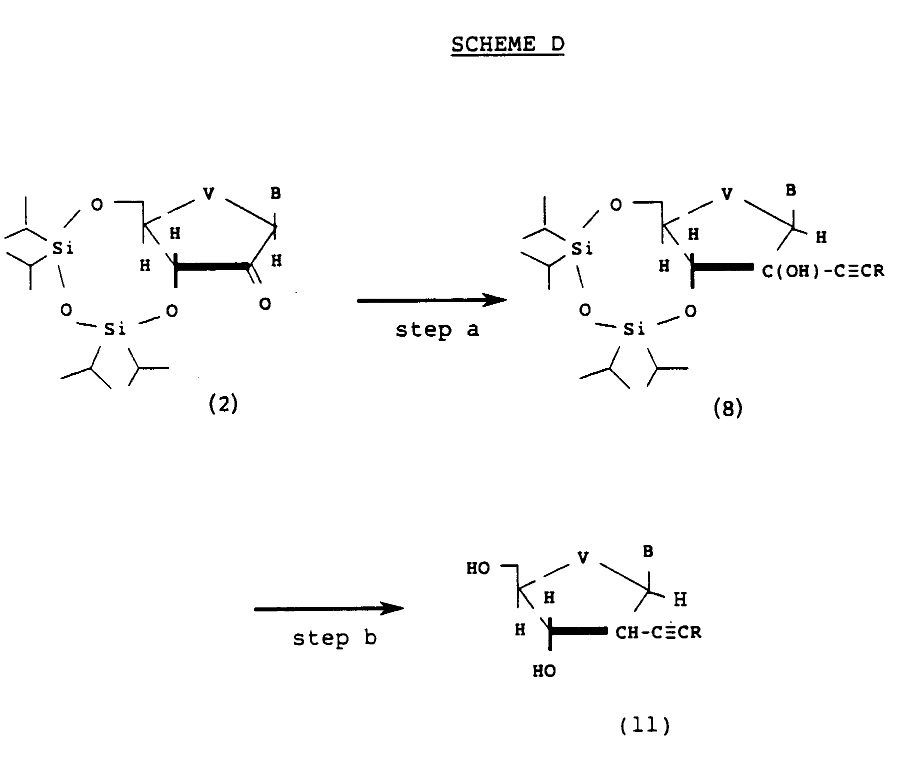
In step a, the ketone derivative (2), for example, 4-ethoxy-1-[(3,5-O-tetraisopropyldisiloxan-1,3-diyl)-β-D-erythro-pentofuran-2-keto-osyl]-2(1H)-pyrimidone, can be reacted as described in Scheme C (step a) to yield the corresponding 2-ethynyl alcohol (8). As described for Scheme C, the 2-ethynyl alcohol (8) can exist as two diasteriomeric isomers, for example, 4-ethoxy-1-[(3,5-O-tetraisopropyldisiloxan-1,3-diyl)-β-D-ribo-pentofuran-2-(ethynyl)osyl]-2(1H)-pyrimidone and 4-ethoxy-1-[(3,5-O-tetraisopropyldisiloxan-1,3-diyl)-β-D-arabino-pentofuran-2-(ethynyl)osyl]-2(1H)-pyrimidone.

In step b, the 2-ethynyl alcohol (8) is reduced and the tetraisopropyldisiloxan blocking group is removed to yield the 2-ethynyl derivative (11). This reduction can be carried out according to methods and procedures which are well known and appreciated in the art of chemistry by treating the 2-ethynyl alcohol (8) with a reducing agent such as triethylsilane in the presence of an acid such as trifluoroacetic acid.

Where a 4-alkoxy-substituted pyrimidone, such as the 4-ethoxy-substituted pyrimidone, is utilized as the starting material (2) for step a, the 4-alkoxy moiety of the pyrimidone base can be converted to the 4-keto moiety to yield the corresponding uridine or thymidine derivative (11) or it can be converted to the 4-amino moiety to yield the corresponding cytidine derivative (11). These reactions can be effected according to procedures well known and appreciated in the art of chemistry as described for Scheme A.

Again it will be appreciated that the 2′-ethynyl derivative (11) can exist as one of two diasteriomeric isomers, i.e., one wherein the ethynyl group is on the same side of the furanosyl ring as the 3-hydroxy group and one wherein the ethynyl group is on the same side of the furanosyl ring as the purine or pyrimidine group. For example, where a cytidine derivative is desired, these geometric isomers can be named 2′-deoxy-2′(R)-ethynylcytidine (or 4-keto-1-[β-D-erythro-pentofuran-2(R)-(ethynyl)osyl]-2(1H)-pyrimidone) and 2′-deoxy-2′(S)-ethynylcytidine (or 4-keto-1-[β-D-erythro-pentofuran-2(S)-(ethynyl)osyl]-2(1H)-pyrimidone), respectively.

As is readily apparent to one skilled in the art, the (R) and the (S) diasteriomeric isomers of the 2′-ethynyl derivatives (11) can be separated using conventional separation methods. For example, the diasteriomeric isomers may be resolved by column chromatography using known methods and techniques.

The following example presents a typical synthesis as described by Scheme D. This example is understood to be illustrative only and is not intended to limit the scope of the present invention in any way.

### EXAMPLE 10

### 2′-DEOXY-2′(R or S)-ETHYNYLCYTIDINE

### Step a: 4-Ethoxy-1-[(3,5-O-tetraisopropyldisiloxan-1,3-diyl)-β-D-(ribo- or arabino-)pentofuran-2-(ethynyl)osyl]-2(1H)-pyrimidone

Prepare the title compound as described in Example 7, step a.

### Step b: 2′-Deoxy-2′(R and S)-ethynylcytidine

Dissolve 4-ethoxy-1-[(3,5-O-tetraisopropyldisiloxan-1,3-diyl)-β-D-(ribo- or arabino-)pentofuran-2-(ethynyl)osyl]-2(1H)-pyrimidone (490 mg, 0.94 mmol) in dichloromethane (3ml) under a nitrogen atmosphere and cool the solution in an ice bath (0°C). Add trifluoroacetic acid (0.54 ml, 7.08 mmol) followed by triethylsilane (0.27 ml, 1.71 mmol) and stir the solution overnight at room temperature. Dilute the reaction mixture with ethyl acetate (10 ml) and wash with ice cold 1N sodium hydroxide solution (2 times, 5 ml each time). Dry the organic layer with anhydrous magnesium sulfate and evaporate to dryness to yield 4-ethoxy-1-[β-D-erythro-pentofuran-2(R and S)-(ethynyl)osyl]-2(1H)-pyrimidone.

Heat a solution of 4-ethoxy-1-[β-D-erythro-pentofuran-2(R and S)-(ethynyl)osyl]-2(1H)-pyrimidone (885 mg, 3 mmol) in methanolic ammonia (10 ml, saturated at 0°C) in a sealed tube at 100°C for 2 days. Evaporate the solution to dryness and separate the (R)- and (S)- isomers on a Dowex 1-X2 (OH⁻ form) chromatographic column to provide the title compounds.

The following compounds can be made by procedures analogous to those described above in Example 10:
2′-deoxy-2′(R or S)-ethynyl-5-methylcytidine
2′-deoxy-2′(R or S)-ethynyl-5-hydroxymethylcytidine
2′-deoxy-2′(R or S)-ethynyl-4′-thiocytidine
(±)-(1β,3α,4β)-1-(4-amino-2-hydroxypyrimidin-6-yl)-2-(α and β)-ethynyl-3-hydroxy-4-hydroxymethylcyclopentane

### EXAMPLE 11

### 2′-DEOXY-2′(R and S)-ETHYNYLURIDINE

### Step a: 4-Ethoxy-1-[(3,5-O-tetraisopropyldisiloxan-1,3-diyl)-β-D-(ribo- or arabino-)pentofuran-2-(ethynyl)osyl]-2(1H)-pyrimidone

Prepare the title compound as described in Example 7, step a.

### Step b: 2′-Deoxy-2′(R and S)-ethynyluridine

Prepare 4-ethoxy-1-[β-D-erythro-pentofuran-2(R and S)-(ethynyl)osyl]-2(1H)-pyrimidone as described in Example 10, step b.

Stir a solution of 4-ethoxy-1-[β-D-erythro-pentofuran-2(R and S)-(ethynyl)osyl]-2(1H)-pyrimidone(590 mg, 2 mmol) in THF (15 ml) and 1N sodium hydroxide (5 ml) at room temperature for 23 hours and then at 60°C for 2 hours. Neutralize the reaction solution with Amberlite IRC-50 (H⁺-form) and filter off the resin. Evaporate the filtrate to dryness and separate the (R)- and (S)- isomers on a Dowex 1-X2 (OH⁻ form) chromatographic column to provide the title compounds.

The following compounds can be made by procedures analogous to those described above in Example 11:
2′-deoxy-2′(R and S)-ethynyl-5-hydroxymethyluridine
2′-deoxy-2′(R and S)-ethynylthymidine
2′-deoxy-2′(R and S)-ethynyl-4′-thiouridine

### EXAMPLE 12

### 2′-DEOXY-2′(R and S)-ETHYNYLGUANOSINE

### Step a: 9-[(3,5-O-tetraisopropyldisiloxan-1,3-diyl)-β-D-(ribo-or arabino-)pentofuran-2-(ethynyl)osyl]guanine

Prepare the title compound from 9-[(3,5-O-tetraisopropyldisiloxan-1,3-diyl)-2-keto-β-D-erythro-pentofuranosyl]guanine (2.58 gm, 5 mmol) as described in Example 7, step a.

### Step b: 2′-Deoxy-2′(R and S)-ethynylguanosine

Prepare the title compouunds from 9-[(3,5-O-tetraisopropyldisiloxan-1,3-diyl)-β-D-(ribo- or arabino-)pentofuran-2-(ethynyl)osyl]guanine as described in Example 10, step b.

The following compounds can be made by procedures analogous to those described above in Example 9:
2′-deoxy-2′(R and S)-ethynyl-4′-thioguanosine
2′-deoxy-2′(R and S)-8-azaguanosine
2′-deoxy-2′(R and S)-N³-methylguanosine
2′-deoxy-2′(R and S)-8-chloroguanosine
In many of the examples above, the 4-ethoxy-pyrimidone derivative is utilized as the starting material (2). It will be appreciated by those skilled in the art that the 4-ethoxy moiety can be converted, if desired, to either a keto group (to form a uridine/thymidine derivative) or to an amino group (to form a cytidine derivative). Alternatively, where a uridine, thymidine or cytidine derivative is desired, a 2-keto starting material (2) can be utilized wherein the base moiety is cytosine, uracil or thymine.

Starting materials for use in the general synthetic procedure outlined in Schemes A through D are readily available by the use of synthetic methods and procedures which are well known and appreciated by those of ordinary skill in the art. For example, 4-ethoxy-1-[(3,5-O-tetraisopropyldisiloxan-1,3-diyl)-2-keto-β-D-erythro-pentofuranosyl]-2(1H)-pyrimidone can be used as the starting material for many of the compounds of formulas (1), (1a) and (1b) and can be prepared from uridine according to the procedure described by Matsuda et al.[ *Chem.Pharm.Bull.* **1988**, 36, 945]. 9-[(3,5-O-Tetraisopropyldisiloxan-1,3-diyl)-2-keto-β-D-erythro-pentofuranosyl]guanine can be prepared from guanosine by a procedure analogous to that described by Hansske et al. [*Tetrahedron* **1984**, 40, 125]. Other 2-keto starting materials can be prepared by the use of methods analogous to those described by the methods described above as well as other conventional methods as are well known and appreciated in the art such as that described by Hansske and Robins [*Tetrahedron Lett.* **1983**, 24, 1589].

In a further embodiment, the present invention provides a method for the treatment of a patient afflicted with a neoplastic disease state comprising the administration thereto of a therapeutically effective antineoplastic amount of a compound of formula (1), (1a) or (1b). The term "neoplastic disease state" as used herein refers to an abnormal state or condition characterized by rapidly proliferating cell growth or neoplasm. Neoplastic disease states for which treatment with a compound of formula (1), (1a) or (1b) will be particularly useful include: Leukemias such as, but not limited to, acute lymphoblastic, chronic lymphocytic, acute myloblastic and chronic mylocytic; Carcinomas, such as, but not limited to, those of the cervix, oesophagus, stomach, small intestines, colon and lungs; Sarcomas, such as, but not limited to, oesteroma, osteosarcoma, lepoma, liposarcoma, hemangioma and hemangiosarcoma; Melanomas, including amelanotic and melanotic; and mixed types of neoplasias such as, but not limited to carcinosarcoma, lymphoid tissue type, folicullar reticulum, cell sarcoma and Hodgkins Disease. Neoplastic disease states for which treatment with a compound of formula (1), (1a) or (1b) will be particularly preferred include:
2′-deoxy-2′-difluoromethylidenecytidine
2′-deoxy-2′-difluoromethylideneuridine
2′-deoxy-2′-difluoromethylideneguanosine
(Z) and (E)-2′-deoxy-2′-fluoromethylidenecytidine
(Z) and (E)-2′-deoxy-2′-fluoromethylideneuridine
(Z) and (E)-2′-deoxy-2′-fluoromethylideneguanosine
2′-deoxy-2′-ethenylidenecytidine
2′-deoxy-2′-ethenylideneuridine
2′-deoxy-2′-ethenylideneguanosine
2′-deoxy-2′(R and S)-ethynylcytidine
2′-deoxy-2′(R and S)-ethynyluridine
2′-deoxy-2′(R and S)-ethynylguanosine
As used herein, the term "patient" refers to a warm-blooded animal, such as a human, which is afflicted with a particular neoplastic or viral disease state.

A therapeutically effective antineoplastic amount of a compound of formula (1), (1a) or (1b) refers to an amount which is effective, upon single or multiple dose administration to the patient, in controlling the growth of the neoplasm or in prolonging the survivability of the patient beyond that expected in the absence of such treatment. As used herein, "controlling the growth" of the neoplasm refers to slowing, interrupting, arresting or stopping its growth and metastases and does not necessarily indicate a total elimination of the neoplasm.

In addition, the present invention provides a method for the treatment of a patient afflicted with a viral infection comprising the administration thereto of a therapeutically effective antiviral amount of a compound of formula (1), (1a) or (1b). The term "viral infection" as used herein refers to an abnormal state or condition characterized by viral transformation of cells, viral replication and proliferation. Viral infections for which treatment with a compound of the formula (1), (1a) or (1b) will be particularly useful include: Retroviruses such as, but not limited to, HTLV-I, HTLV-II, human immunodeficiency viruses, HTLV-III (AIDS virus), and the like; RNA viruses such as, but not limited to, influenza type A, B, and C, mumps, measles, rhinovirus, dengue, rubella, rabies, hepatitis virus A, encephalitis virus, and the like; DNA viruses such as, but not limited to, herpes, vaccinia, pappiloma virus (wart), hepatitis virus B, and the like.

A "therapeutically effective antiviral amount" of a compound of formula (1), (1a) or (1b) refers to an amount which is effective, upon single or multiple dose administration to the patient, in controlling the growth of the virus or in prolonging the survivability of the patient beyond that expected in the absence of such treatment. As used herein, "controlling the growth" of the virus refers to slowing, interrupting, arresting or stopping the viral transformation of cells or the replication and proliferation of the virus and does not necessarily indicate a total elimination of the virus.

As used herein, the term "therapeutically effective amount" refers to a therapeutically effective antineoplastic or antiviral amount of a compound of the formula (1), (1a) or (1b). A therapeutically effective amount can be readily determined by the attending diagnostician, as one skilled in the art, by the use of known techniques and by observing results obtained under analogous circumstances. In determining the therapeutically effective amount or dose, a number of factors are considered by the attending diagnostician, including, but not limited to: the species of mammal; its size, age, and general health; the specific disease involved; the degree of or involvement or the severity of the disease; the response of the individual patient; the particular compound administered; the mode of administration; the bioavailability characteristics of the preparation administered; the dose regimen selected; the use of concomitant medication; and other relevant circumstances.

A therapeutically effective amount of a compound of formula (1), (1a) or (1b) is expected to vary from about 0.1 milligram per kilogram of body weight per day (mg/kg/day) to about 100 mg/kg/day. Preferred amounts are expected to vary from about 0.5 to about 10 mg/kg/day.

In effecting treatment of a patient afflicted with a disease state described above, a compound of formula (1), (1a) or (1b) can be administered in any form or mode which makes the compound bioavailable in effective amounts, including oral and parenteral routes. For example, compounds of formula (1), (1a) or (1b) can be administered orally, subcutaneously, intramuscularly, intravenously, transdermally, intranasally, rectally, and the like. Oral administration is generally preferred. One skilled in the art of preparing formulations can readily select the proper form and mode of administration depending upon the particular characteristics of the compound selected the disease state to be treated, the stage of the disease, and other relevant circumstances.

The compounds can be administered alone or in the form of a pharmaceutical composition in combination with pharmaceutically acceptable carriers or excipients, the proportion and nature of which are determined by the solubility and chemical properties of the compound selected, the chosen route of administration, and standard pharmaceutical practice. The compounds of the invention, while effective themselves, may be formulated and administered in the form of their pharmaceutically acceptable acid addition salts for purposes of stability, convenience of crystallization, increased solubility and the like.

In another embodiment, the present invention provides compositions comprising a compound of formula (1), (1a) or (1b) in admixture or otherwise in association with one or more inert carriers. These compositions are useful, for example, as assay standards, as convenient means of making bulk shipments, or as pharmaceutical compositions. An assayable amount of a compound of formula (1), (1a) or (1b) is an amount which is readily measurable by standard assay procedures and techniques as are well known and appreciated by those skilled in the art. Assayable amounts of a compound of formula (1), (1a) or (1b) will generally vary from about 0.001% to about 75% of the composition by weight. Inert carriers can be any material which does not degrade or otherwise covalently react with a compound of formula (1), (1a) or (1b). Examples of suitable inert carriers are water; aqueous buffers, such as those which are generally useful in High Performance Liquid Chromatography (HPLC) analysis; organic solvents, such as acetonitrile, ethyl acetate, hexane and the like; and pharmaceutically acceptable carriers or excipients.

More particularly, the present invention provides pharmaceutical compositions comprising a therapeutically effective amount of a compound of formula (1), (1a) or (1b) in admixture or otherwise in association with one or more pharmaceutically acceptable carriers or excipients.

The pharmaceutical compositions are prepared in a manner well known in the pharmaceutical art. The carrier or excipient may be a solid, semi-solid, or liquid material which can serve as a vehicle or medium for the active ingredient. Suitable carriers or excipients are well known in the art. The pharmaceutical composition may be adapted for oral or parenteral use and may be administered to the patient in the form of tablets, capsules, suppositories, solution, suspensions, or the like.

The compounds of the present invention may be administered orally, for example, with an inert diluent or with an edible carrier. They may be enclosed in gelatin capsules or compressed into tablets. For the purpose of oral therapeutic administration, the compounds may be incorporated with excipients and used in the form of tablets, troches, capsules, elixirs, suspensions, syrups, wafers, chewing gums and the like. These preparations should contain at least 4% of the compound of the invention, the active ingredient, but may be varied depending upon the particular form and may conveniently be between 4% to about 70% of the weight of the unit. The amount of the compound present in compositions is such that a suitable dosage will be obtained. Preferred compositions and preparations according to the present invention are prepared so that an oral dosage unit form contains between 5.0-300 milligrams of a compound of the invention.

The tablets, pills, capsules, troches and the like may also contain one or more of the following adjuvants: binders such as microcrystalline cellulose, gum tragacanth or gelatin; excipients such as starch or lactose, disintegrating agents such as alginic acid, Primogel, corn starch and the like; lubricants such as magnesium stearate or Sterotex; glidants such as colloidal silicon dioxide; and sweetening agents such as sucrose or saccharin may be added or a flavoring agent such as peppermint, methyl salicylate or orange flavoring. When the dosage unit form is a capsule, it may contain, in addition to materials of the above type, a liquid carrier such as polyethylene glycol or a fatty oil. Other dosage unit forms may contain other various materials which modify the physical form of the dosage unit, for example, as coatings. Thus, tablets or pills may be coated with sugar, shellac, or other enteric coating agents. A syrup may contain, in addition to the present compounds, sucrose as a sweetening agent and certain preservatives, dyes and colorings and flavors. Materials used in preparing these various compositions should be pharmaceutically pure and non-toxic in the amounts used.

For the purpose of parenteral therapeutic administration, the compounds of the present invention may be incorporated into a solution or suspension. These preparations should contain at least 0.1% of a compound of the invention, but may be varied to be between 0.1 and about 50% of the weight thereof. The amount of the inventive compound present in such compositions is such that a suitable dosage will be obtained. Preferred compositions and preparations according to the present invention are prepared so that a parenteral dosage unit contains between 5.0 to 100 milligrams of the compound of the invention.

The solutions or suspensions may also include the one or more of the following adjuvants: sterile diluents such as water for injection, saline solution, fixed oils, polyethylene glycols, glycerine, propylene glycol or other synthetic solvents; antibacterial agents such as benzyl alcohol or methyl paraben; antioxidants such as ascorbic acid or sodium bisulfite; chelating agents such as ethylene diaminetetraacetic acid; buffers such as acetates, citrates or phosphates and agents for the adjustment of tonicity such as sodium chloride or dextrose. The parenteral preparation can be enclosed in ampules, disposable syringes or multiple dose vials made of glass or plastic.

As with any group of structurally related compounds which possesses a particular generic utility, certain groups and configurations are preferred for compounds of formula (1), (1a) or (1b) in their end-use application.

With respect to the substituents X₁ and X₂, compounds of formula (1) wherein X₁ is fluoro and X₂ is hydrogen, and those wherein X₁ is hydrogen and X₂ is fluoro, are generally preferred.

With respect to the substituent R, compounds of the formulas (1a) and (1b) wherein R is hydrogen are generally preferred.

The following are additional preferred embodiments for compounds of formula (1), (1a) or (1b): compounds wherein V is oxy, compounds wherein Y₁ is a CH group, compounds wherein Y₂ is nitrogen, compounds wherein Y₃ is nitrogen and compounds wherein Z is hydrogen are generally preferred.

The following list identifies compounds of the formula (1),(1a) and (1b) which are particularly preferred embodiments of the present invention:
2′-deoxy-2′-difluoromethylidene-cytidine
2′-deoxy-2′-difluoromethylidene-uridine
2′-deoxy-2′-difluoromethylidene-guanosine
(Z) and (E) 2′-deoxy-2′-fluoromethylidene-cytidine
(Z) and (E) 2′-deoxy-2′-fluoromethylidene-uridine
(Z) and (E) 2-deoxy-2′-fluoromethylidene-guanosine
2′-deoxy-2′-ethenylidene-cytidine
2′-deoxy-2′-ethenylidene-uridine
2′-deoxy-2′(R) and (S)-ethynyl-cytidine
2′-deoxy-2′(R) and (S)-ethynyl-uridine
2′-deoxy-2′(R) and (S)-ethynyl-guanosine
The above list is intended to be merely illustrative of particularly preferred embodiments of the present invention and it is understood that the list does not limit the scope of the invention in any way.

## Claims (Claims for the following Contracting State(s): AT, BE, CH, DE, FR, GB, IT, LI, LU, NL, SE)

1. A compound of the formula 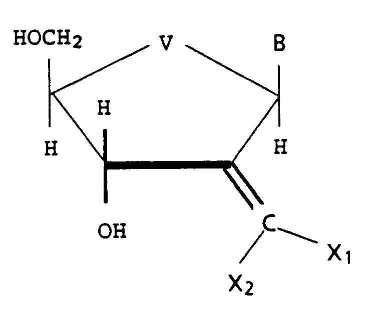 wherein
V is oxy, methylene, or thio,
X₁ and X₂ are each independently hydrogen or halogen,
with the proviso that at least one of X₁ and X₂ is halogen,
B is a radical of the formula 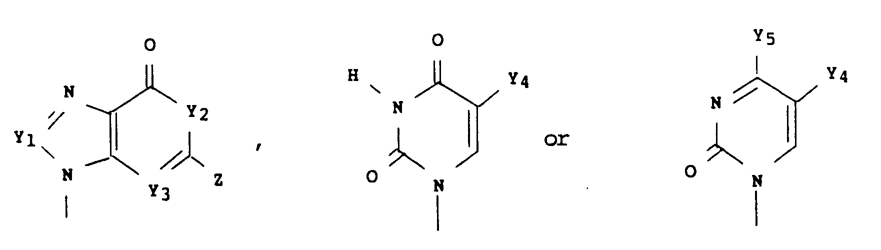 wherein Y₁ is nitrogen, a CH group, a CCl group, a CBr group or a CNH₂ group; Y₂ and Y₃ are each independently nitrogen or a CH group; Y₄ is hydrogen, C₁-C₄ alkyl, C₁-C₄ alkoxy or halogen; Y₅ is amino or C₁-C₄ alkoxy; and Z is hydrogen, halogen, or NH₂;
or a pharmaceutically acceptable salt thereof.

2. A compound of the formula 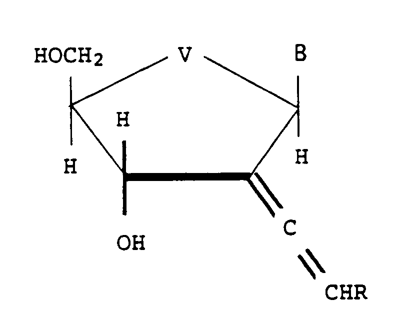 wherein
V is oxy, methylene, or thio,
R is hydrogen or C₁-C₄ alkyl,
B is a radical of the formula 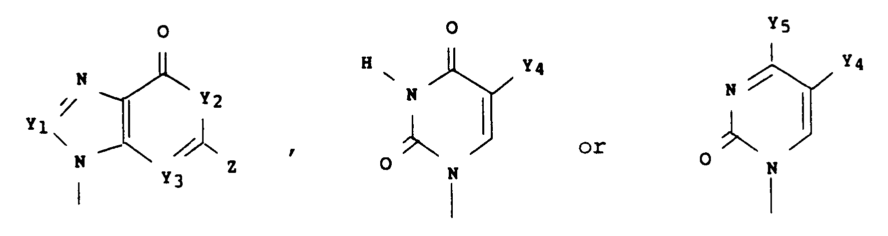 wherein Y₁ is nitrogen, a CH group, a CCl group, a CBr group or a CNH₂ group; Y₂ and Y₃ are each independently nitrogen or a CH group; Y₄ is hydrogen, C₁-C₄ alkyl, C₁-C₄ alkoxy or halogen; Y₅ is amino or C₁-C₄ alkoxy; and Z is hydrogen, halogen, or NH₂;
or a pharmaceutically acceptable salt thereof.

3. A compound of the formula 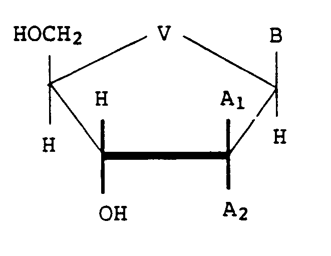 wherein
V is oxy, methylene, or thio,
A₁ and A₂ are each independently hydrogen or a -C≡CR group, wherein R is hydrogen or C₁-C₄ alkyl, with the proviso that where A₁ is hydrogen A₂ is a -C≡CR group, and where A₁ is a -C≡CR group A₂ is hydrogen,
B is a radical of the formula 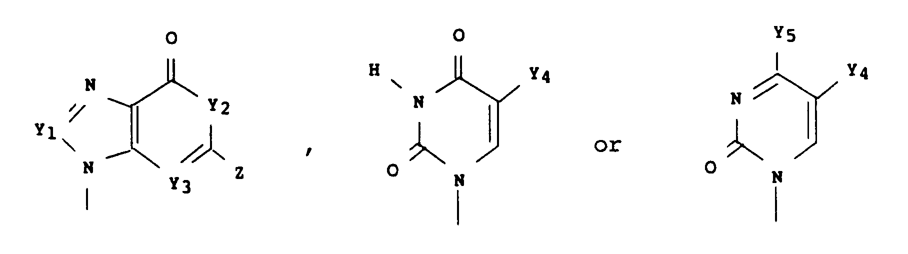 wherein Y₁ is nitrogen, a CH group, a CCl group, a CBr group or a CNH₂ group; Y₂ and Y₃ are each independently nitrogen or a CH group; Y₄ is hydrogen, C₁-C₄ alkyl, C₁-C₄ alkoxy or halogen; Y₅ is amino or C₁-C₄ alkoxy; and Z is hydrogen, halogen, or NH₂;
or a pharmaceutically acceptable salt thereof.

4. A compound of Claim 1 wherein X₁ is fluoro and X₂ is hydrogen.

5. A compound of claim 1 wherein X₁ is hydrogen and X₂ is fluoro.

6. A compound of claim 2 or 3 wherein R is hydrogen.

7. A compound of claim 1, 2 or 3 wherein V is oxy.

8. A compound of claim 1, 2 or 3 wherein Z is hydrogen.

9. A compound of claim 1, 2 or 3 wherein Y₁ is a CH group.

10. A compound of claim 1, 2 or 3 wherein Y₂ is nitrogen.

11. A compound of claim 1, 2, or 3 wherein Y₃ is nitrogen.

12. A compound of claim 1, 2 or 3 wherein Z is amino.

13. A compound of claim 1, 2 or 3 wherein Y₂ is a CH group.

14. A compound of claim 1 wherein the compound is 2′-deoxy-2′-difluoromethylidene-cytidine.

15. The compound of claim 1 wherein the compound is 2′-deoxy-2′-difluoromethylidene-uridine.

16. The compound of claim 1 wherein the compound is 2′-deoxy-2′-difluoromethylidene-guanosine.

17. A compound of claim 1 wherein the compound is (Z) or (E)-2′-deoxy-2′-fluoromethylidene-cytidine.

18. A compound of claim 1 wherein the compound is (Z) or (E)-2′-deoxy-2′-fluoromethylidene-uridine.

19. A compound of claim 1 wherein the compound is (Z) or (E)-2′-deoxy-2′-fluoromethylidene-guanosine.

20. The compound of claim 2 wherein the compound is 2′-deoxy-2′-ethenylidene-cytidine.

21. The compound of claim 2 wherein the compound is 2′-deoxy-2′-ethenylidene-uridine.

22. The compound of claim 2 wherein the compound is 2′-deoxy-2′-ethenylidene-guanosine.

23. A compound of claim 3 wherein the compound is 2′-deoxy-2′(R or S)-ethynyl-cytidine.

24. A compound of claim 3 wherein the compound is 2′-deoxy-2′(R or S)-ethynyl-uridine.

25. A compound of claim 3 wherein the compound is 2′-deoxy-2′(R or S)-ethynyl-guanosine.

26. A compound of the formula 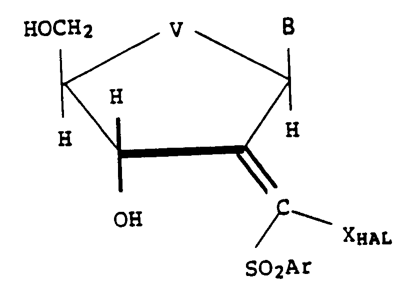 wherein
V is oxy, methylene or thio,
X_{HAL} is halogen,
Ar is a C₆-C₁₂ aryl group,
B is a radical of the formula 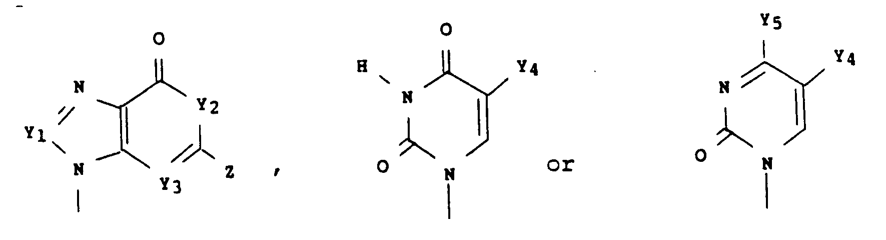 wherein Y₁ is nitrogen, a CH group, a CCl group, a CBr group or a CNH₂ group; Y₂ and Y₃ are each independently nitrogen or a CH group; Y₄ is hydrogen, C₁-C₄ alkyl, C₁-C₄ alkoxy or halogen, Y₅ is amino or C₁-C₄, alkoxy; and Z is hydrogen, halogen or NH₂.

27. A compound of claim 26 wherein Ar is phenyl.

28. A compound of claim 27 wherein X_{HAL} is fluorine.

29. A compound of any of claims 1 to 25 for use as a medicament.

30. A compound of any of claims 1 to 25 for use as a medicament for the treatment of a neoplastic disease.

31. A compound of any claims 1 to 25 for use as a medicament for treatment of viral infections.

32. Use of a compound of any claims 1 to 25 for the manufacture of a medicament for treatment of a neoplastic disease or viral infections.

33. A pharmaceutical composition containing a compound of claims 1 to 25 as the active ingredient in admixture with one or more pharmaceutically acceptable carriers or excipients.

## Claims (Claims for the following Contracting State(s): GR, ES)

1. A process for making a compound of the formula 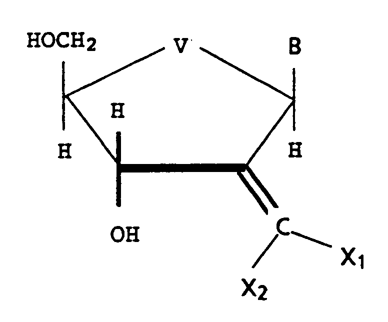 wherein
V is oxy, methylene or thio,
X₁ and X₂ are each halogen,
B is a radical of the formula 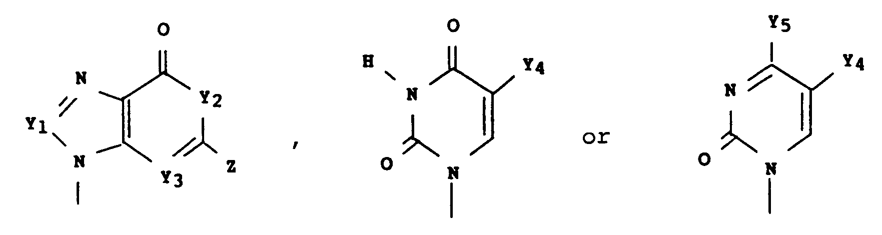 wherein Y₁ is nitrogen, a CH group, a CCl group, a CBr group or a CNH₂ group; Y₂ and Y₃ are each independently nitrogen or a CH group; Y₄ is hydrogen, C₁-C₄ alkyl, C₁-C₄ alkoxy or halogen; Y₅ is amino or C₁-C₄ alkoxy; and Z is hydrogen, halogen, or NH₂;
or a pharmaceutically acceptable salt thereof, comprising the steps of
a) reacting a 3,5-O-tetraisopropyldisiloxan-1,3-diyl-2-ketonucleoside derivative with a dihalomethylidene phosphorus ylide to yield a 3,5-O-tetraisopropyldisiloxan-1,3-diyl-2-dihalomethylidene-nucleoside derivative,
b) optionally reacting the 3,5-O-tetraisopropyldisiloxan-1,3-diyl-2-dihalomethylidene-nucleoside derivative with base, and
c) reacting the 3,5-O-tetraisopropyldisiloxan-1,3-diyl-2-dihalomethylidene-nucleoside derivative with a fluoride anion or acid.

2. A process for making a compound of the formula 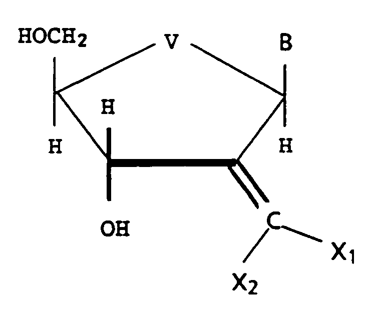 wherein
V is oxy, methylene, or thio,
One of X₁ and X₂ is halogen and the other is hydrogen,
B is a radical of the formula 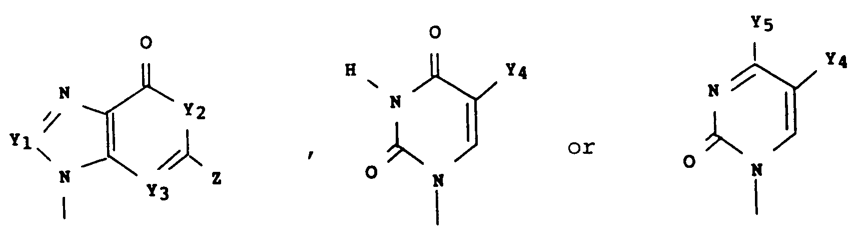 wherein Y₁ is nitrogen, a CH group, a CCl group, a CBr group or a CNH₂ group; Y₂ and Y₃ are each independently nitrogen or a CH group; Y₄ is hydrogen, C₁-C₄ alkyl, C₁-C₄ alkoxy or halogen; Y₅ is amino or C₁-C₄ alkoxy; and Z is hydrogen, halogen, or NH₂;
or a pharmaceutically acceptable salt thereof, comprising the steps of
a) reacting a 3,5-O-tetraisopropyldisiloxan-1,3-diyl-2-keto-nucleoside derivative with an arylsufonylhalo methylidene phosphorus ylide to yield a 3,5-O-tetra isopropyldisiloxan-1,3-diyl-2-arylsulfonylhalomethylidene-nucleoside derivative,
b) reacting the 3,5-O-tetraisopropyldisiloxan-1,3-diyl-2-arylsulfonylhalomethylidene-nucleoside derivative with a fluoride anion or acid to yield the corresponding 2-arylsulfonylhalomethylidene-nucleoside derivative,
c) reacting the 2-arylsulfonylhalomethylidene-nucleoside derivative with an aluminum/mercury amalgam, a sodium/mercury amalgam, or tributyltinhydride/azobisiso butylnitrile followed by aqueous acid treatment, and
d) optionally reacting the 2-arylsulfonylhalomethylidene-nucleoside derivative with base.

3. A process for making a compound of the formula 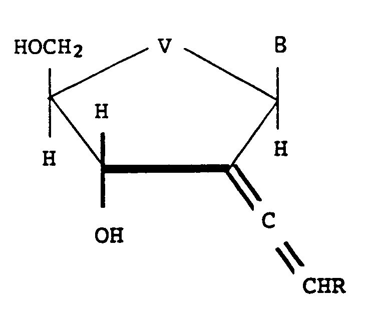 wherein
V is oxy, methylene, or thio,
R is hydrogen or C₁-C₄ alkyl,
B is a radical of the formula 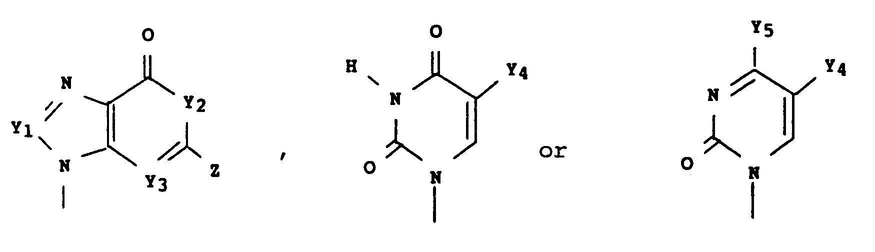 wherein Y₁ is nitrogen, a CH group, a CCl group, a CBr group or a CNH₂ group; Y₂ and Y₃ are each independently nitrogen or a CH group; Y₄ is hydrogen, C₁-C₄ alkyl, C₁-C₄ alkoxy or halogen; Y₅ is amino or C₁-C₄ alkoxy; and Z is hydrogen, halogen, or NH₂;
or a pharmaceutically acceptable salt thereof, comprising the steps of
a) reacting a 3,5-O-tetraisopropyldisiloxan-1,3-diyl-2-ketonucleoside derivative with an acetylenic Grignard reagent to yield a 3,5-O-tetraisopropyldisiloxan-1,3-diyl-2-ethynyl-2-hydroxy-nucleoside derivative,
b) reacting the 3,5-O-tetraisopropyldisiloxan-1,3-diyl-2-ethynyl-2-hydroxy-nucleoside derivative with a reducing agent such as lithium aluminum hydride and aluminum chloride to yield the corresponding 3,5-O-tetraisopropyldisiloxan-1,3-diyl-2-ethenylidene-nucleoside derivative,
c) optionally reacting the 3,5-O-tetraisopropyldisiloxan-1,3-diyl-2-ethenylidene-nucleoside derivative with base, and
d) reacting the 3,5-O-tetraisopropyldisiloxan-1,3-diyl-2-ethenylidene-nucleoside derivative with a fluoride anion or acid.

4. A process for making a compound of the formula 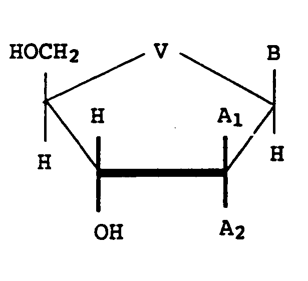 wherein
V is oxy, methylene, or thio,
A₁ and A₂ are each independently hydrogen or a -C≡CR group, wherein R is hydrogen or C₁-C₄ alkyl, with the proviso that where A₁ is hydrogen A₂ is a -C≡CR group, and where A₁ is a -C≡CR group A₂ is hydrogen,
B is a radical of the formula 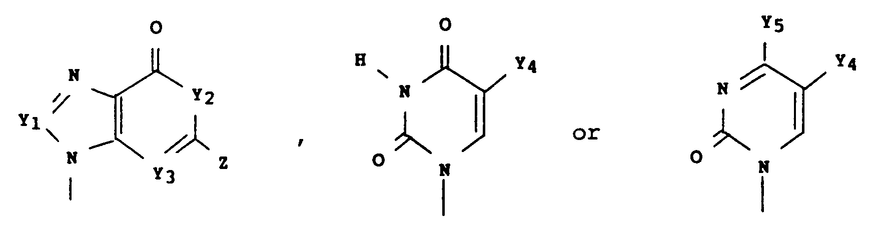 wherein Y₁ is nitrogen, a CH group, a CCl group, a CBr group or a CNH₂ group; Y₂ and Y₃ are each independently nitrogen or a CH group; Y₄ is hydrogen, C₁-C₄ alkyl, C₁-C₄ alkoxy or halogen; Y₅ is amino or C₁-C₄ alkoxy; and Z is hydrogen, halogen, or NH₂;
or a pharmaceutically acceptable salt thereof, comprising the steps of
a) reacting a 3,5-O-tetraisopropyldisiloxan-1,3-diyl-2-keto-nucleoside derivative with an acetylenic Grignard reagent to yield a 3,5-O-tetraisopropyldisiloxan-1,3-diyl-2-ethynyl-2-hydroxy-nucleoside derivative,
b) reacting the 3,5-O-tetraisopropyldisiloxan-1,3-diyl-2-ethynyl-2-hydroxy-nucleoside derivative with a reducing agent such as triethylsilane to yield the corresponding 3,5-O-tetraisopropyldisiloxan-1,3-diyl-2-ethynyl-nucleoside derivative,
c) optionally reacting the 3,5-O-tetraisopropyldisiloxan-1,3-diyl-2-ethynyl-nucleoside derivative with base, and
d) reacting the 3,5-O-tetraisopropyldisiloxan-1,3-diyl-2-ethynyl-nucleoside derivative with fluoride anion or acid.

5. A process for making a compound of the formula 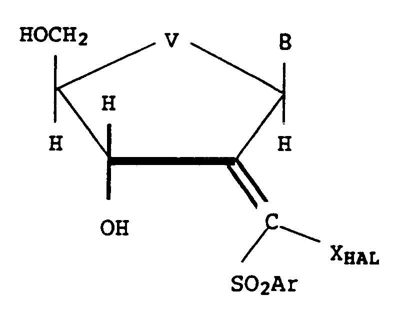 wherein
V is oxy, methylene, or thio,
X_{HAL} is halogen,
Ar is a C₆-C₁₂ aryl group,
B is a radical of the formula 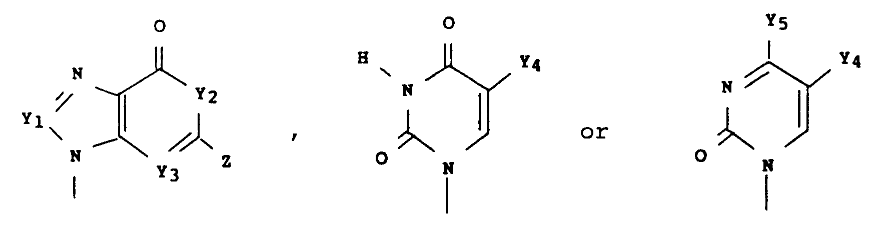 wherein Y₁ is nitrogen, a CH group, a CCl group, a CBr group or a CNH₂ group; Y₂ and Y₃ are each independently nitrogen or a CH group; Y₄ is hydrogen, C₁-C₄ alkyl, C₁-C₄ alkoxy or halogen; Y₅ is amino or C₁-C₄ alkoxy; and Z is hydrogen, halogen, or NH₂;
or a pharmaceutically acceptable salt thereof, comprising the steps of
a) reacting a 3,5-O-tetraisopropyldisiloxan-1,3-diyl-2-keto-nucleoside derivative with an arylsufonylhalo methylidene phosphorus ylide to yield a 3,5-O-tetra isopropyldisiloxan-1,3-diyl-2-arylsulfonylhalomethylidene-nucleoside derivative,
b) reacting the 3,5-O-tetraisopropyldisiloxan-1,3-diyl-2-arylsulfonylhalomethylidene-nucleoside derivative with a fluoride anion or acid to yield the corresponding 2-arylsulfonylhalomethylidene-nucleoside derivative, and
c) optionally reacting the 2-arylsulfonylhalomethylidene-nucleoside derivative with base.

6. A process according to claim 2 for preparing a compound wherein X₁ is fluoro and X₂ is hydrogen.

7. A process according to claim 2 for preparing a compound wherin X₁ is hydrogen and X₂ is fluoro.

8. A process according to any of claims 3 and 4 for preparing a compound wherein R is hydrogen.

9. A process according to any of claims 1 to 4 for preparing a compound wherein V is oxy.

10. A process according to any of claims 1 to 4 for preparing a compound wherein Z is hydrogen.

11. A process according to any of claims 1 to 4 for preparing a compound wherein Y₁ is a CH group.

12. A process according to any of claims 1 to 4 for preparing a compound wherein Y₂ is nitrogen.

13. A process according to any of claims 1 to 4 for preparing a compound wherein Y₃ is nitrogen.

14. A process according to any of claims 1 to 4 for preparing a compound wherein Z is amino.

15. A process according to any of claims 1 to 4 for preparing a compound wherein Y₂ is a CH group.

16. A process according to claim 1 for preparing a compound that is 2′-deoxy-2′-difluoromethylidene-cytidine.

17. A process according to claim 1 for preparing a compound that is 2′-deoxy-2′-difluoromethylidene-uridine.

18. A process according to claim 1 for preparing a compound that is 2′-deoxy-2′-difluoromethylidene-guanosine.

19. A process according to claim 2 for preparing a compound that is (Z) or (E)-2′-deoxy-2′-fluoromethylidene-cytidine.

20. A process according to claim 2 for preparing a compound that is (Z) or (E)-2′-deoxy-2′-fluoromethylidene-uridine.

21. A process according to claim 2 for preparing a compound that is (Z) or (E)-2′-deoxy-2′-fluoromethylidene-guanosine.

22. A process according to claim 3 for preparing a compound that is 2′-deoxy-2′-ethenylidene-cytidine.

23. A process according to claim 3 for preparing a compound that is 2′-deoxy-2′-ethenylidene-uridine.

24. A process according to claim 3 for preparing a compound that is 2′-deoxy-2′-ethenylidene-guanosine.

25. A process according to claim 4 for preparing a compound that is 2′-deoxy-2′(R or S)-ethynyl-cytidine.

26. A process according to claim 4 for preparing a compound that is 2′-deoxy-2′(R or S)-ethynyl-uridine.

27. A process according to claim 4 for preparing a compound that is 2′-deoxy-2′(R or S)-ethynyl-guanosine.

28. A process according to claim 5 for preparing a compound wherein Ar is phenyl.

29. A process according to claim 5 for preparing a compound wherein Ar is phenyl and X_{HAL} is fluorine.

## Patentansprüche (Patentansprüche für folgende(n) Vertragsstaat(en): AT, BE, CH, DE, FR, GB, IT, LI, LU, NL, SE)

1. Verbindung der Formel 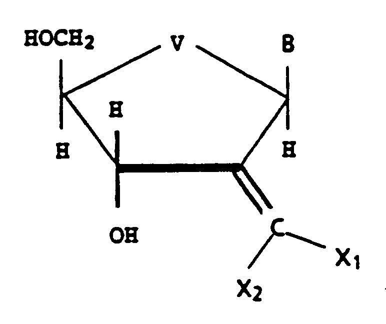 in der
V eine Oxy-, Methylen- oder Thiogruppe bedeutet,
X₁ und X₂ jeweils unabhängig voneinander ein Wasserstoff- oder Halogenatom bedeuten, mit der Maßgabe, daß mindestens einer der Reste X₁ und X₂ ein Halogenatom bedeutet,
B eine Gruppe der Formel 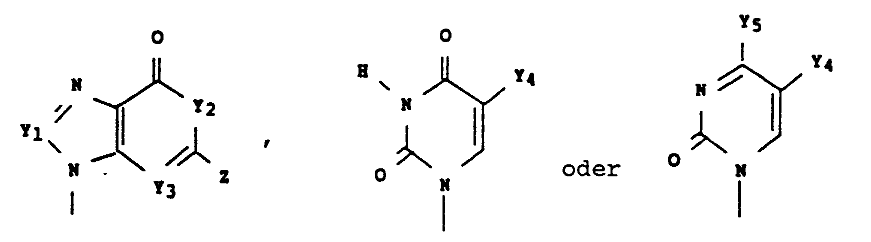 bedeutet, wobei Y₁ ein Stickstoffatom, eine CH-Gruppe, eine CCl-Gruppe, eine CBr-Gruppe oder eine CNH₂-Gruppe bedeutet, Y₂ und Y₃ jeweils unabhängig voneinander ein Stickstoffatom oder eine CH-Gruppe darstellen, Y₄ ein Wasserstoffatom, einen C₁-C₄-Alkyl- oder C₁-C₄-Alkoxyrest oder ein Halogenatom darstellt; Y₅ eine Aminogruppe oder einen C₁-C₄-Alkoxyrest bedeutet, und Z ein Wasserstoff- oder Halogenatom oder die Gruppe NH₂ darstellt;
oder ein pharmazeutisch verträgliches Salz davon.

2. Verbindung der Formel 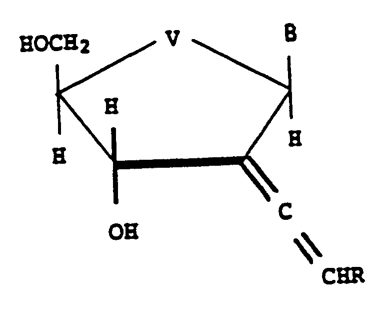 in der
V eine Oxy-, Methylen- oder Thiogruppe bedeutet,
R ein Wasserstoffatom oder einen C₁-C₄-Alkylrest darstellt,
B eine Gruppe der Formel 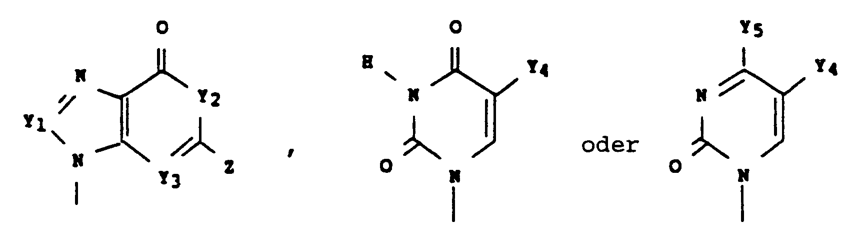 bedeutet, wobei Y₁ ein Stickstoffatom, eine CH-Gruppe, eine CCl-Gruppe, eine CBr-Gruppe oder eine CNH₂-Gruppe bedeutet, Y₂ und Y₃ jeweils unabhängig voneinander ein Stickstoffatom oder eine CH-Gruppe darstellen, Y₄ ein Wasserstoffatom, einen C₁-C₄-Alkyl- oder C₁-C₄-Alkoxyrest oder ein Halogenatom bedeutet, Y₅ eine Aminogruppe oder einen C₁-C₄-Alkoxyrest darstellt und Z ein Wasserstoff- oder Halogenatom oder die Gruppe NH₂ darstellt;
oder ein pharmazeutisch verträgliches Salz davon.

3. Verbindung der Formel 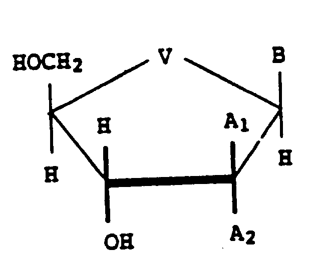 in der
V eine Oxy-, Methylen- oder Thiogruppe bedeutet,
A₁ und A₂ jeweils unabhängig voneinander ein Wasserstoffatom oder eine -C≡CR-Gruppe darstellen, wobei R ein Wasserstoffatom oder einen C₁-C₄-Alkylrest bedeutet, mit der Maßgabe, daß, wenn A₁ ein Wasserstoffatom bedeutet,
A₂ eine -C≡CR-Gruppe ist und wenn A₁ eine -C≡CR-Gruppe bedeutet, A₂ ein Wasserstoffatom ist,
B eine Gruppe der Formel 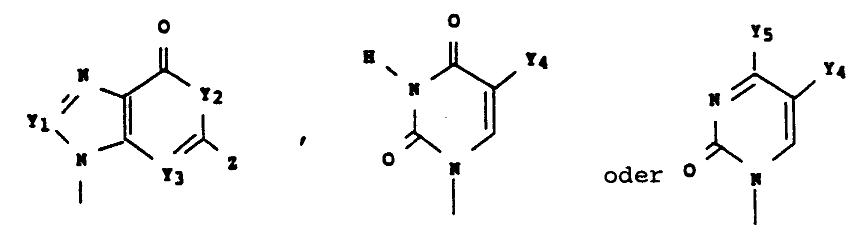 bedeutet, wobei Y₁ ein Stickstoffatom, eine CH-Gruppe, eine CCl-Gruppe, eine CBr-Gruppe oder eine CNH₂-Gruppe bedeutet, Y₂ und Y₃ jeweils unabhängig voneinander ein Stickstoffatom oder eine CH-Gruppe darstellen, Y₄ ein Wasserstoffatom, einen C₁-C₄-Alkyl- oder C₁-C₄-Alkoxyrest oder ein Halogenatom bedeutet, Y₅ eine Aminogruppe oder einen C₁-C₄-Alkoxyrest darstellt und Z ein Wasserstoff- oder Halogenatom oder die Gruppe NH₂ darstellt;
oder ein pharmazeutisch verträgliches Salz davon.

4. Verbindung nach Anspruch 1, wobei X₁ ein Fluoratom und X₂ ein Wasserstoffatom bedeutet.

5. Verbindung nach Anspruch 1, wobei X₁ ein Wasserstoffatom und X₂ ein Fluoratom bedeutet.

6. Verbindung nach Anspruch 2 oder 3, wobei R ein Wasserstoffatom bedeutet.

7. Verbindung nach Anspruch 1, 2 oder 3, wobei V eine Oxygruppe darstellt.

8. Verbindung nach Anspruch 1, 2 oder 3, wobei Z ein Wasserstoffatom bedeutet.

9. Verbindung nach Anspruch 1, 2 oder 3, wobei Y₁ eine CH-Gruppe darstellt.

10. Verbindung nach Anspruch 1, 2 oder 3, wobei Y₂ ein Stickstoffatom darstellt.

11. Verbindung nach Anspruch 1, 2 oder 3, wobei Y₃ ein Stickstoffatom darstellt.

12. Verbindung nach Anspruch 1, 2 oder 3, wobei Z eine Aminogruppe darstellt.

13. Verbindung nach Anspruch 1, 2 oder 3, wobei Y₂ eine CH-Gruppe bedeutet.

14. Verbindung nach Anspruch 1, nämlich 2'-Desoxy-2'-difluormethyliden-cytidin.

15. Verbindung nach Anspruch 1, nämlich 2'-Desoxy-2'-difluormethyliden-uridin.

16. Verbindung nach Anspruch 1, nämlich 2'-Desoxy-2'-difluormethyliden-guanosin.

17. Verbindung nach Anspruch 1, nämlich (Z) oder (E)-2'-Desoxy-2'-fluormethyliden-cytidin.

18. Verbindung nach Anspruch 1, nämlich (Z) oder (E)-2'-Desoxy-2'-fluormethyliden-uridin.

19. Verbindung nach Anspruch 1, nämlich (Z) oder (E)-2'-Desoxy-2'-fluormethyliden-guanosin.

20. Verbindung nach Anspruch 2, nämlich 2'-Desoxy-2'-ethenyliden-cytidin.

21. Verbindung nach Anspruch 2, nämlich 2'-Desoxy-2'-ethenyliden-uridin.

22. Verbindung nach Anspruch 2, nämlich 2'-Desoxy-2'-ethenyliden-guanosin.

23. Verbindung nach Anspruch 3, nämlich 2'-Desoxy-2'(R oder S)-ethinyl-cytidin.

24. Verbindung nach Anspruch 3, nämlich 2'-Desoxy-2'(R oder S)-ethinyl-uridin.

25. Verbindung nach Anspruch 3, nämlich 2'-Desoxy-2'(R oder S)-ethinyl-guanosin.

26. Verbindung nach der Formel 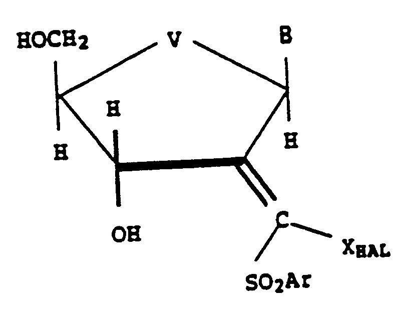 in der
V eine Oxy-, Methylen- oder Thiogruppe bedeutet,
X_{HAL} ein Halogenatom darstellt,
Ar eine C₆-C₁₂-Arylgruppe bedeutet,
B eine Gruppe der Formel 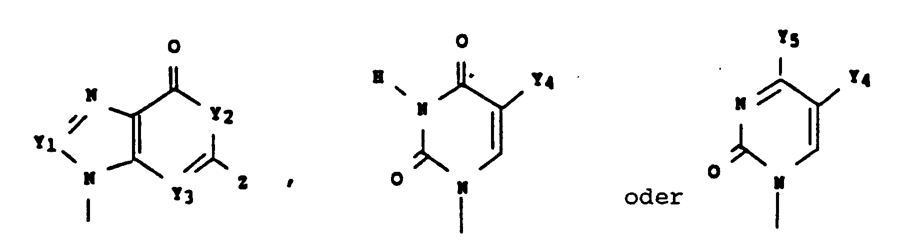 darstellt, wobei Y₁ ein Stickstoffatom, eine CH-Gruppe, eine CCl-Gruppe, eine CBr-Gruppe oder eine CNH₂-Gruppe bedeutet, Y₂ und Y₃ jeweils unabhängig voneinander ein Stickstoffatom oder eine CH-Gruppe darstellen, Y₄ ein Wasserstoffatom, einen C₁-C₄-Alkyl- oder C₁-C₄-Alkoxyrest oder ein Halogenatom bedeutet, Y₅ eine Aminogruppe oder einen C₁-C₄-Alkoxyrest bedeutet und Z ein Wasserstoff- oder Halogenatom oder die Gruppe NH₂ bedeutet.

27. Verbindung nach Anspruch 26, wobei Ar eine Phenylgruppe bedeutet.

28. Verbindung nach Anspruch 27, wobei X_{HAL} ein Fluoratom darstellt.

29. Verbindung nach einem der Ansprüche 1 bis 25 zur Verwendung als Arzneimittel.

30. Verbindung nach einem der Ansprüche 1 bis 25 zur Verwendung als Arzneimittel zur Behandlung einer neoplastischen Erkrankung.

31. Verbindung nach einem der Ansprüche 1 bis 25 zur Verwendung als Arzneimittel zur Behandlung viraler Infektionen.

32. Verwendung einer Verbindung nach einem der Ansprüche 1 bis 25 zur Herstellung eines Arzneimittels zur Behandlung einer neoplastischen Erkrankung oder viralen Infektionen.

33. Arzneimittel, enthaltend eine Verbindung nach den Ansprüchen 1 bis 25 als Wirkstoff im Gemisch mit einem oder mehreren pharmazeutisch verträglichen Trägern oder Exzipienten.

## Patentansprüche (Patentansprüche für folgende(n) Vertragsstaat(en): GR, ES)

1. Verfahren zur Herstellung einer Verbindung der Formel 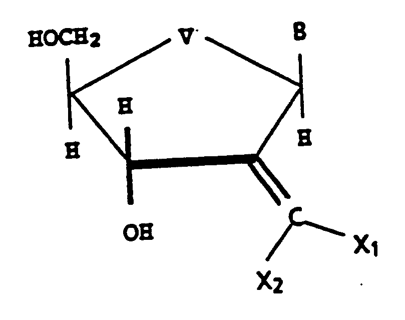 in der
V eine Oxy-, Methylen- oder Thiogruppe bedeutet,
X₁ und X₂ jeweils unabhängig voneinander ein Halogenatom bedeuten,
B eine Gruppe der Formel 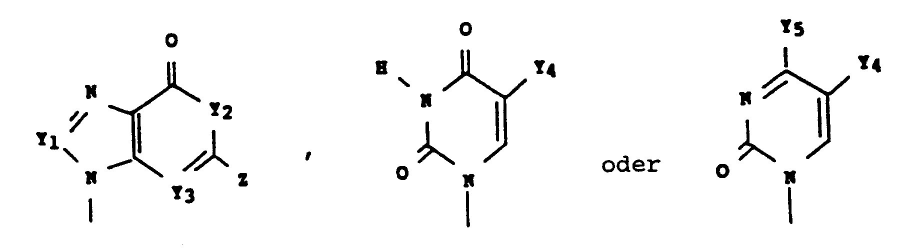 bedeutet, wobei Y₁ ein Stickstoffatom, eine CH-Gruppe, eine CCl-Gruppe, eine CBr-Gruppe oder eine CNH₂-Gruppe bedeutet, Y₂ und Y₃ jeweils unabhängig voneinander ein Stickstoffatom oder eine CH-Gruppe darstellen, Y₄ ein Wasserstoffatom, einen C₁-C₄-Alkyl- oder C₁-C₄-Alkoxyrest oder ein Halogenatom darstellt; Y₅ eine Aminogruppe oder einen C₁-C₄-Alkoxyrest bedeutet, und Z ein Wasserstoff- oder Halogenatom oder die Gruppe NH₂ darstellt;
oder eines pharmazeutisch verträglichen Salzes davon, umfassend die Schritte
a) Umsetzung eines 3,5-O-Tetraisopropyldisiloxan-1,3-diyl-2-ketonucleosidderivats mit einem Dihalogenmethylidenphosphorylid zu einem 3,5-O-Tetraisopropyldisiloxan-1,3-diyl-2-dihalogenmethylidennucleosidderivat,
b) gegebenenfalls Umsetzen des 3,5-O-Tetraisopropyldisiloxan-1,3-diyl-2-dihalogenmethylidennucleosidderivats mit einer Base, und
c) Umsetzen des 3,5-O-Tetraisopropyldisiloxan-1,3-diyl-2-dihalogenmethylidennucleosidderivats mit einem Fluoridanion oder einer Säure.

2. Verfahren zur Herstellung einer Verbindung der Formel 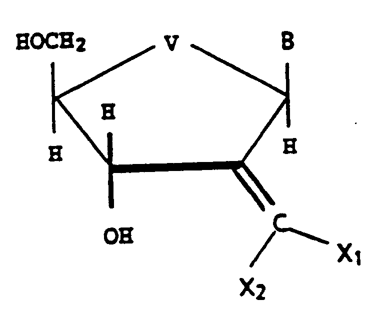 in der
V eine Oxy-, Methylen- oder Thiogruppe bedeutet,
einer der Reste X₁ und X₂ ein Halogenatom bedeutet und der andere ein Wasserstoffatom darstellt,
B eine Gruppe der Formel 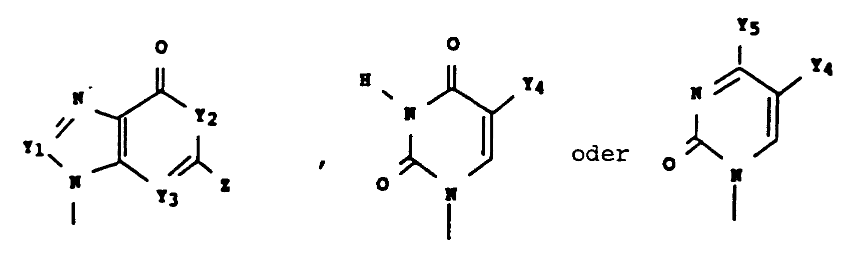 bedeutet, wobei Y₁ ein Stickstoffatom, eine CH-Gruppe, eine CCl-Gruppe, eine CBr-Gruppe oder eine CNH₂-Gruppe bedeutet, Y₂ und Y₃ jeweils unabhängig voneinander ein Stickstoffatom oder eine CH-Gruppe darstellen, Y₄ ein Wasserstoffatom, einen C₁-C₄-Alkyl- oder C₁-C₄-Alkoxyrest oder ein Halogenatom bedeutet, Y₅ eine Aminogruppe oder einen C₁-C₄-Alkoxyrest darstellt und Z ein Wasserstoff- oder Halogenatom oder die Gruppe NH₂ darstellt;
oder eines pharmazeutisch verträglichen Salzes davon, umfassend die Schritte
a) Umsetzung eines 3,5-O-Tetraisopropyldisiloxan-1,3-diyl-2-ketonucleosidderivats mit einem Arylsulfonylhalogenmethylenphosphorylid zu einem 3,5-O-Tetraisopropyldisiloxan-1,3-diyl-2-arylsulfonylhalogenmethylidennucleosidderivat,
b) Umsetzen des 3,5-O-Tetraisopropyldisiloxan-1,3-diyl-2-arylsulfonylhalogenmethylidennucleosidderivats mit einem Fluoridanion oder einer Säure zu dem entsprechenden 2-Arylsulfonylhalogenmethylidennucleosidderivat,
c) Umsetzen des 2-Arylsulfonylhalogenmethylidennucleosidderivats mit einem Aluminium/Quecksilberamalgam, Natrium/Quecksilberamalgam oder Tributylzinnhydrid/Azobisisobutylnitril, gefolgt von einer Behandlung mit wäßriger Säure und
d) gegebenenfalls Umsetzen des 2-Arylsulfonylhalogenmethylidennucleosidderivats mit einer Base.

3. Verfahren zur Herstellung einer Verbindung der Formel 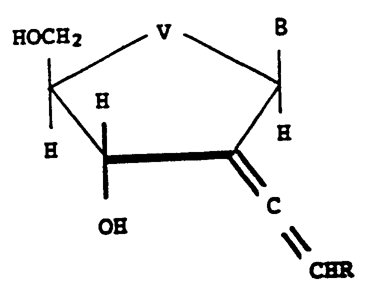 in der
V eine Oxy-, Methylen- oder Thiogruppe bedeutet,
R ein Wasserstoffatom oder einen C₁-C₄-Alkylrest bedeutet,
B eine Gruppe der Formel 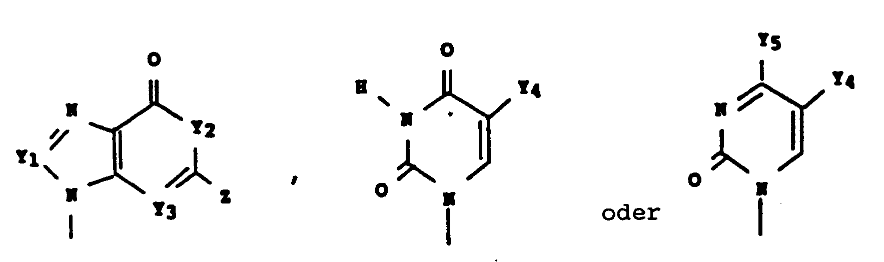 bedeutet, wobei Y₁ ein Stickstoffatom, eine CH-Gruppe, eine CCl-Gruppe, eine CBr-Gruppe oder eine CNH₂-Gruppe bedeutet, Y₂ und Y₃ jeweils unabhängig voneinander ein Stickstoffatom oder eine CH-Gruppe darstellen, Y₄ ein Wasserstoffatom, einen C₁-C₄-Alkyl- oder C₁-C₄-Alkoxyrest oder ein Halogenatom bedeutet, Y₅ eine Aminogruppe oder einen C₁-C₄-Alkoxyrest darstellt und Z ein Wasserstoff- oder Halogenatom oder die Gruppe NH₂ darstellt;
oder eines pharmazeutisch verträglichen Salzes davon, umfassend die Schritte
a) Umsetzen eines 3,5-O-Tetraisopropyldisiloxan-1,3-diyl-2-ketonucleosidderivats mit einem acetylenischen Grignardreagenz zu einem 3,5-O-Tetraisopropyldisiloxan-1,3-diyl-2-ethinyl-2-hydroxynucleosidderivat,
b) Umsetzen des 3,5-O-Tetraisopropyldisiloxan-1,3-diyl-2-ethinyl-2-hydroxynucleosidderivats mit einem Reduktionsmittel, wie Lithiumaluminiumhydrid und Aluminiumchlorid zu dem entsprechenden 3,5-O-Tetraisopropyldisiloxan-1,3-diyl-2-ethenylidennucleosidderivat,
c) gegebenenfalls Umsetzen des 3,5-O-Tetraisopropyldisiloxan-1,3-diyl-2-ethenylidennucleosidderivats mit einer Base und
d) Umsetzen des 3,5-O-Tetraisopropyldisiloxan-1,3-diyl-2-ethenyliden-nucleosidderivats mit einem Fluoridanion oder Säure.

4. Verfahren zur Herstellung einer Verbindung der Formel 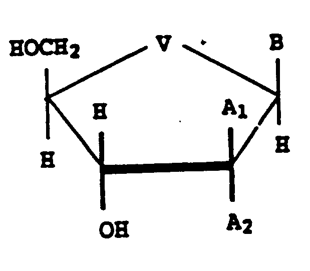 in der
V eine Oxy-, Methylen- oder Thiogruppe bedeutet,
A₁ und A₂ jeweils unabhängig voneinander ein Wasserstoffatom oder eine -C≡CR-Gruppe darstellen, wobei R ein Wasserstoffatom oder ein C₁-C₄-Alkylrest bedeutet, mit der Maßgabe, daß, wenn A₁ ein Wasserstoffatom bedeutet, A₂ eine -C≡CR-Gruppe ist und wenn A₁ eine -C≡CR-Gruppe bedeutet, A₂ ein Wasserstoffatom ist,
B eine Gruppe der Formel 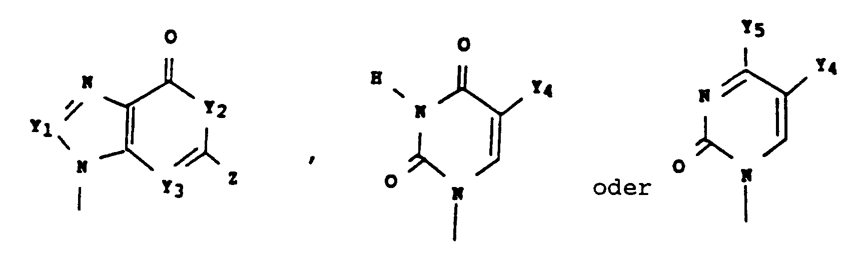 bedeutet, wobei Y₁ ein Stickstoffatom, eine CH-Gruppe, eine CCl-Gruppe, eine CBr-Gruppe oder eine CNH₂-Gruppe bedeutet, Y₂ und Y₃ jeweils unabhängig voneinander ein Stickstoffatom oder eine CH-Gruppe darstellen, Y₄ ein Wasserstoffatom, einen C₁-C₄-Alkyl- oder C₁-C₄-Alkoxyrest oder ein Halogenatom bedeutet, Y₅ eine Aminogruppe oder einen C₁-C₄-Alkoxyrest darstellt und Z ein Wasserstoff- oder Halogenatom oder die Gruppe NH₂ darstellt;
oder eines pharmazeutisch verträglichen Salzes davon, umfassend die Schritte
a) Umsetzen eines 3,5-O-Tetraisopropyldisiloxan-1,3-diyl-2-ketonucleosidderivats mit einem acetylenischen Grignardreagenz zu einem 3,5-O-Tetraisopropyldisiloxan-1,3-diyl-2-ethinyl-2-hydroxynucleosidderivat,
b) Umsetzen des 3,5-O-Tetraisopropyldisiloxan-1,3-diyl-2-ethinyl-2-hydroxynucleosidderivat mit einem Reduktionsmittel, wie Triethylsilan zu dem entsprechenden 3,5-O-Tetraisopropyldisiloxan-1,3-diyl-2-ethinyl-nucleosidderivat,
c) gegebenenfalls Umsetzung des 3,5-O-Tetraisopropyldisiloxan-1,3-diyl-2-ethinylnucleosidderivats mit einer Base und
d) Umsetzen des 3,5-O-Tetraisopropyldisiloxan-1,3-diyl-2-ethinylnucleosidderivats mit einem Fluoridanion oder Säure.

5. Verfahren zur Herstellung einer Verbindung der Formel in der 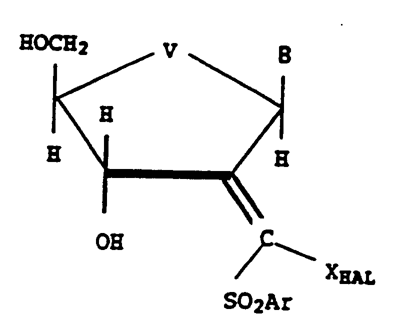
V eine Oxy-, Methylen- oder Thiogruppe bedeutet,
X_{HAL}, ein Halogenatom darstellt,
Ar eine C₆-C₁₂-Arylgruppe darstellt,
B einen Rest der Formel 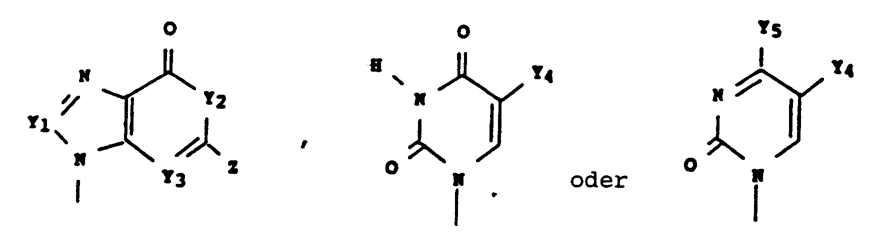 bedeutet, wobei Y₁ ein Stickstoffatom, eine CH-Gruppe, eine CCl-Gruppe, eine CBr-Gruppe oder eine CNH₂-Gruppe bedeutet, Y₂ und Y₃ jeweils unabhängig voneinander ein Stickstoffatom oder eine CH-Gruppe darstellen, Y₄ ein Wasserstoffatom, einen C₁-C₄-Alkyl- oder C₁-C₄-Alkoxyrest oder ein Halogenatom bedeutet, Y₅ eine Aminogruppe oder einen C₁-C₄-Alkoxyrest darstellt und Z ein Wasserstoff- oder Halogenatom oder die Gruppe NH₂ bedeutet,
oder eines pharmazeutisch verträglichen Salzes davon, umfassend die Schritte
a) Umsetzen eines 3,5-O-Tetraisopropyldisiloxan-1,3-diyl-2-ketonucleosidderivats mit einem Arylsulfonylhalogenmethylidenphosphorylid zu einem 3,5-O-Tetraisopropyldisiloxan-1,3-diyl-2-arylsulfonylhalogenmethylidennucleosidderivat,
b) Umsetzen des 3,5-O-Tetraisopropyldisiloxan-1,3-diyl-2-arylsulfonylhalogenmethylidennucleosidderivats mit einem Fluoridanion oder einer Säure zu dem entsprechenden 2-Arylsulfonylhalogenmethylidennucleosidderivat und
c) gegebenenfalls Umsetzung des 2-Arylsulfonylhalogenmethylidennucleosidderivats mit einer Base.

6. Verfahren nach Anspruch 2 zur Herstellung einer Verbindung, wobei X₁ ein Fluoratom bedeutet und X₂ ein Wasserstoffatom bedeutet.

7. Verfahren nach Anspruch 2 zur Herstellung einer Verbindung, wobei X₁ ein Wasserstoffatom bedeutet und X₂ ein Fluoratom bedeutet.

8. Verfahren nach einem der Ansprüche 3 und 4 zur Herstellung einer Verbindung, wobei R ein Wasserstoffatom bedeutet.

9. Verfahren nach einem der Ansprüche 1 bis 4 zur Herstellung einer Verbindung, wobei V eine Oxygruppe darstellt.

10. Verfahren nach einem der Ansprüche 1 bis 4 zur Herstellung einer Verbindung, wobei Z ein Wasserstoffatom bedeutet.

11. Verfahren nach einem der Ansprüche 1 bis 4 zur Herstellung einer Verbindung, wobei Y₁ eine CH-Gruppe darstellt.

12. Verfahren nach einem der Ansprüche 1 bis 4 zur Herstellung einer Verbindung, wobei Y₂ ein Stickstoffatom darstellt.

13. Verfahren nach einem der Ansprüche 1 bis 4 zur Herstellung einer Verbindung, wobei Y₃ ein Stickstoffatom darstellt.

14. Verfahren nach einem der Ansprüche 1 bis 4 zur Herstellung einer Verbindung, wobei Z eine Aminogruppe darstellt.

15. Verfahren nach einem der Ansprüche 1 bis 4 zur Herstellung einer Verbindung, wobei Y₂ eine CH-Gruppe bedeutet.

16. Verfahren nach Anspruch 1 zur Herstellung einer Verbindung, nämlich 2'-Desoxy-2'-difluormethyliden-cytidin.

17. Verfahren nach Anspruch 1 zur Herstellung einer Verbindung, nämlich 2'-Desoxy-2'-difluormethyliden-uridin.

18. Verfahren nach Anspruch 1 zur Herstellung einer Verbindung, nämlich 2'-Desoxy-2'-difluormethyliden-guanosin.

19. Verfahren nach Anspruch 2 zur Herstellung einer Verbindung, nämlich (Z) oder (E)-2'-Desoxy-2'-fluormethyliden-cytidin.

20. Verfahren nach Anspruch 2 zur Herstellung einer Verbindung, nämlich (Z) oder (E)-2'-Desoxy-2'-fluormethyliden-uridin.

21. Verfahren nach Anspruch 2 zur Herstellung einer Verbindung, nämlich (Z) oder (E)-2'-Desoxy-2'-fluormethyliden-guanosin.

22. Verfahren nach Anspruch 3 zur Herstellung einer Verbindung, nämlich 2'-Desoxy-2'-ethenyliden-cytidin.

23. Verfahren nach Anspruch 3 zur Herstellung einer Verbindung, nämlich 2'-Desoxy-2'-ethenyliden-uridin.

24. Verfahren nach Anspruch 3 zur Herstellung einer Verbindung, nämlich 2'-Desoxy-2'-ethenyliden-guanosin.

25. Verfahren nach Anspruch 4 zur Herstellung einer Verbindung, nämlich 2'-Desoxy-2'(R oder S)-ethinyl-cytidin.

26. Verfahren nach Anspruch 4, zur Herstellung einer Verbindung, nämlich 2'-Desoxy-2'(R oder S)-ethinyl-uridin.

27. Verfahren nach Anspruch 4 zur Herstellung einer Verbindung, nämlich 2'-Desoxy-2'(R oder S)-ethinyl-guanosin.

28. Verfahren nach Anspruch 5 zur Herstellung einer Verbindung, wobei Ar eine Phenylgruppe bedeutet.

29. Verfahren nach Anspruch 5 zur Herstellung einer Verbindung, wobei Ar eine Phenylgruppe darstellt und X_{HAL} ein Fluoratom bedeutet.

## Revendications (Revendications pour l'(les) Etat(s) contractant(s) suivant(s): AT, BE, CH, DE, FR, GB, IT, LI, LU, NL, SE)

1. Un composé de formule 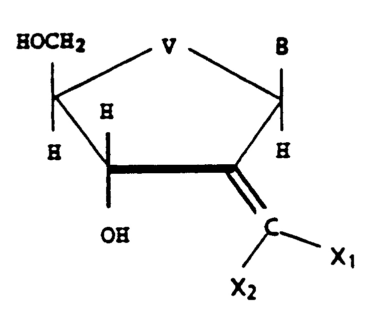 dans laquelle :
V est un groupe oxy, méthylène, ou thio,
X₁ et X₂ représentent chacun indépendamment l'hydrogène ou un halogène, à la condition que l'un au moins des X₁ et X₂ soit un atome d'halogène,
B est un radical de formule 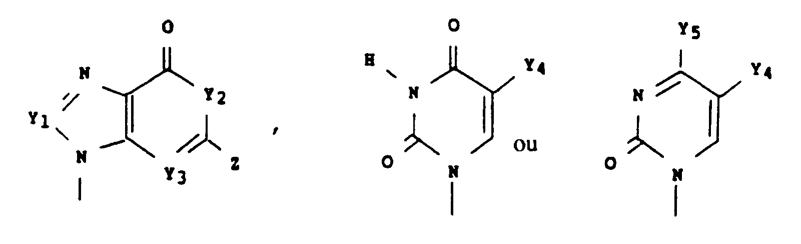 dans laquelle Y₁ est un atome d'azote, un groupe CH, un groupe CCl, un groupe CBr ou un groupe CNH₂ ; Y₂ et Y₃ désignent chacun indépendamment un atome d'azote ou un groupe CH ; Y₄ est l'hydrogène, un groupe C₁-C₄ alkyle, C₁-C₄ alcoxy ou un atome d'halogène ; Y₅ est un groupe amino ou un groupe C₁-C₄ alcoxy ; et Z est l'hydrogène, un atome d'halogène, ou NH₂ ;
ou un sel pharmaceutiquement acceptable de ce composé.

2. Un composé de formule 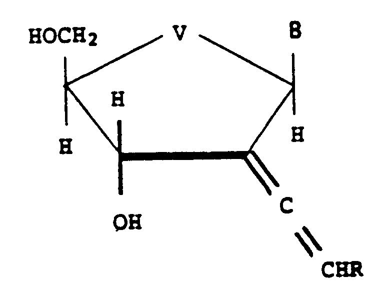 dans laquelle
V est un groupe oxy, méthylène, ou thio,
R est l'hydrogène ou un groupe C₁-C₄ alkyle,
B est un radical de formule 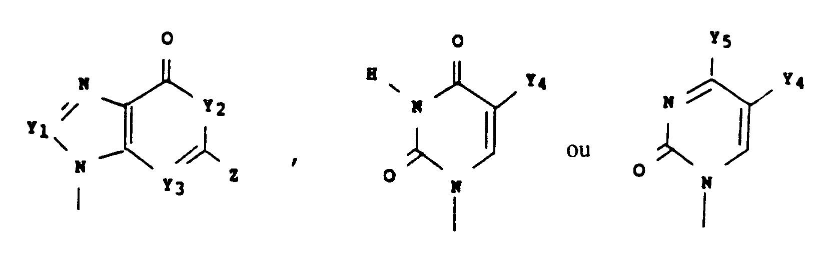 dans laquelle Y₁ est un atome d'azote, un groupe CH, un groupe CCl, un groupe CBr ou un groupe CNH₂ ; Y₂ et Y₃ désignent chacun indépendamment un atome d'azote ou un groupe CH ; Y₄ est l'hydrogène, un groupe C₁-C₄ alkyle, C₁-C₄ alcoxy ou un atome d'halogène ; Y₅ est un groupe amino ou un groupe C₁-C₄ alcoxy ; et Z est l'hydrogène, un atome d'halogène ou un groupe NH₂ ; ou un sel pharmaceutiquement acceptable de ce composé.

3. Un composé de formule 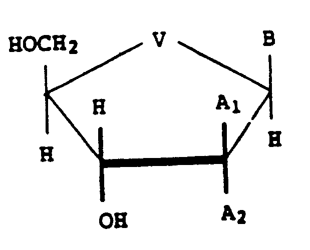 dans laquelle
V est un groupe oxy, méthylène ou thio,
A₁ et A₂ désignent chacun indépendamment l'hydrogène ou un groupe -C≡CR, dans lequel R est l'hydrogène ou un groupe C₁-C₄ alkyle à la condition que lorsque A₁ est l'hydrogène A₂ est un groupe -C≡CR, et lorsque A₁ est un groupe -C≡CR A₂ est l'hydrogène,
B est un radical de formule 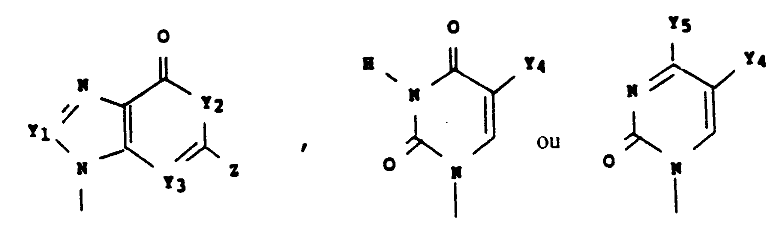 dans laquelle Y₁ est un atome d'azote, un groupe CH, un groupe CCl, un groupe CBr ou un groupe CNH₂ ; Y₂ et Y₃ désignent chacun indépendamment un atome d'azote ou un groupe CH ; Y₄ est l'hydrogène, un groupe C₁-C₄ alkyle, C₁-C₄ alcoxy ou un atome d'halogène ; Y₅ est un groupe amino ou un groupe C₁-C₄ alcoxy ; et Z est l'hydrogène, un atome d'halogène ou un groupe NH₂ ;
ou un sel pharmaceutiquement acceptable de ce composé.

4. Un composé selon la revendication 1, selon laquelle X₁ est un groupe fluoro et X₂ est un atome d'hydrogène.

5. Un composé selon la revendication 1, selon laquelle X₁ est un atome d'hydrogène et X₂ est un groupe fluoro.

6. Un composé selon la revendication 2 ou 3, selon laquelle R est un atome d'hydrogène.

7. Un composé selon la revendication 1, 2 ou 3, selon laquelle V est un groupe oxy.

8. Un composé selon la revendication 1, 2 ou 3, selon laquelle Z est un atome d'hydrogène.

9. Un composé selon la revendication 1, 2 ou 3, selon laquelle Y₁ est un groupe CH.

10. Un composé selon la revendication 1, 2 ou 3, selon laquelle Y₂ est un atome d'azote.

11. Un composé selon la revendication 1, 2 ou 3, selon laquelle Y₃ est un atome d'azote.

12. Un composé selon la revendication 1, 2 ou 3, selon laquelle Z est un groupe amino.

13. Un composé selon la revendication 1, 2 ou 3 selon laquelle Y₂ est un groupe CH.

14. Un composé selon la revendication 1, selon laquelle le composé est la 2'-désoxy-2'-difluorométhylidène-cytidine.

15. Un composé selon la revendication 1, selon laquelle le composé est la 2'-désoxy-2'-difluorométhylidène-uridine.

16. Un composé selon la revendication 1, selon laquelle le composé est la 2'-désoxy-2'-difluorométhylidène-guanosine.

17. Un composé selon la revendication 1, selon laquelle le composé est la (Z) ou la (E)-2'-désoxy-2'-fluorométhylidène-cytidine.

18. Un composé selon la revendication 1, selon laquelle le composé est la (Z) ou la (E)-2'-désoxy,-2-fluorométhylidène-uridine.

19. Un composé selon la revendication 1, selon laquelle le composé est la (Z) ou (E)-2'-désoxy-2'-fluorométhylidène-guanosine.

20. Un composé selon la revendication 2, selon laquelle le composé est la 2'-désoxy-2'-éthénylidène-cytidine.

21. Un composé selon la revendication 2, selon laquelle le composé est la 2'-désoxy-2'-éthénylidène-uridine.

22. Un composé selon la revendication 2, selon laquelle le composé est la 2'-désoxy-2'-éthénylidène-guanosine.

23. Un composé selon la revendication 3, selon laquelle le composé est la 2'-désoxy-2'(R ou S)-éthynyl-cytidine.

24. Un composé selon la revendication 3, selon laquelle le composé est la 2'-désoxy-2'(R ou S)-éthynyl-uridine.

25. Un composé selon la revendication 3, selon laquelle le composé est la 2'-désoxy-2'(R ou S)-éthynyl-guanosine.

26. Un composé de formule dans laquelle 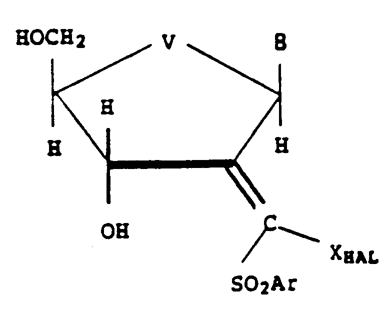
V est un groupe oxy, méthylène ou thio,
X_{HAL} est un atome d'halogène,
Ar est un groupe C₆-C₁₂ aryle,
B est un radical de formule 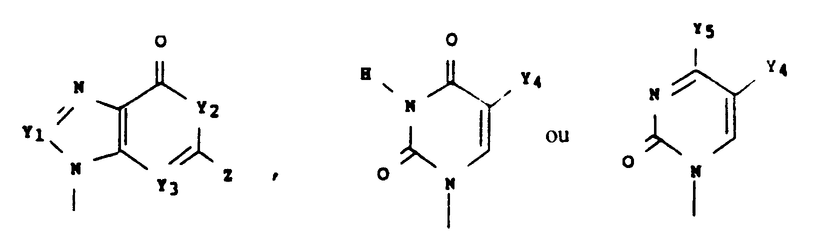 dans laquelle Y₁ est un atome d'azote, un groupe CH, un groupe CCl, un groupe CBr ou un groupe CNH₂ ; Y₂ et Y₃ désignent chacun indépendamment un atome d'azote ou un groupe CH ; Y₂ est l'hydrogène, un groupe C₁-C₄ alkyle, C₁-C₄ alcoxy ou un atome d'halogène, Y₅ est un groupe amino ou un groupe C₁-C₄ alcoxy ; et Z est l'hydrogène, un atome d'halogène ou un groupe NH₂.

27. Un composé selon la revendication 26, selon laquelle Ar est un groupe phényle.

28. Un composé selon la revendication 27, selon laquelle X_{HAL} est du fluor.

29. Un composé selon l'une quelconque des revendications 1 à 25, à utiliser comme médicament.

30. Un composé selon l'une quelconque des revendications 1 à 25, à utiliser comme un médicament pour le traitement d'une maladie néoplasique.

31. Un composé selon l'une quelconque des revendications 1 à 25, à utiliser comme un médicament pour le traitement des infections virales.

32. Utilisation d'un composé selon l'une quelconque des revendications 1 à 25, pour la fabrication d'un médicament pour le traitement d'une maladie néoplasique ou des infections virales.

33. Une composition pharmaceutique contenant un composé selon la revendication 1 à 25, comme ingrédient actif en mélange avec un ou plusieurs supports ou excipients pharmaceutiquement acceptables.

## Revendications (Revendications pour l'(les) Etat(s) contractant(s) suivant(s): GR, ES)

1. Un procédé de fabrication d'un composé de formule 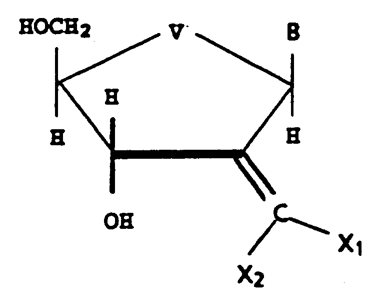 dans laquelle
V est un groupe oxy, méthylène, ou thio,
X₁ et X₂ désignent chacun un atome d'halogène,
B est un radical de formule 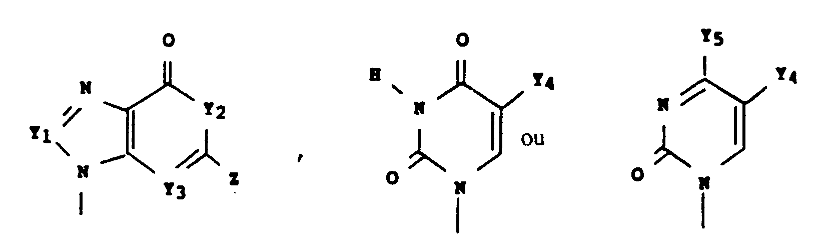 dans laquelle Y₁ est un atome d'azote, un groupe CH, un groupe CCl, un groupe CBr ou un groupe CNH₂ ; Y₂ et Y₃ désignent chacun indépendamment un atome d'azote ou un groupe CH ; Y₄ est l'hydrogène, un groupe C₁-C₄ alkyle, C₁-C₄ alcoxy ou un atome d'halogène ; Y₅ est un groupe amino ou un groupe C₁-C₄ alcoxy ; et Z est l'hydrogène, un atome d'halogène, ou un groupe NH₂ ;
ou d'un sel pharmaceutiquement acceptable de ce composé, comprenant les étapes suivantes :
a) réaction d'un dérivé de 3,5-O-tétraisopropyldisiloxane-1,3-diyl-2-céto-nucléoside avec un dihalométhylidène phosphore ylide pour donner un dérivé de 3,5-O-tétraisopropyldisiloxane-1,3-diyl-2-dihalométhylidène-nucléoside,
b) éventuellement réaction du dérivé de 3,5-O-tétraisopropyldisiloxane-1,3-diyl-2-dihalométhylidène-nucléoside avec une base et
c) réaction du dérivé de 3,5-O-tétraisopropyldisiloxane-1,3-diyl-2-dihalométhylidène-nucléoside avec un anion fluorure ou un acide.

2. Un procédé pour fabriquer un composé de formule 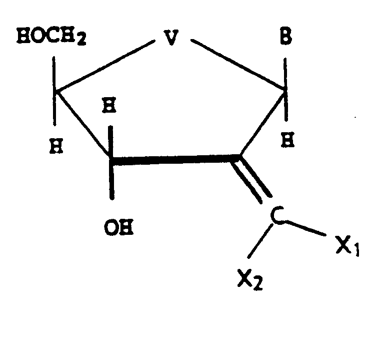 dans laquelle
V est un groupe oxy, méthylène, ou thio,
l'un des X₁ et X₂ est un atome d'halogène et l'autre est l'hydrogène,
B est un radical de formule 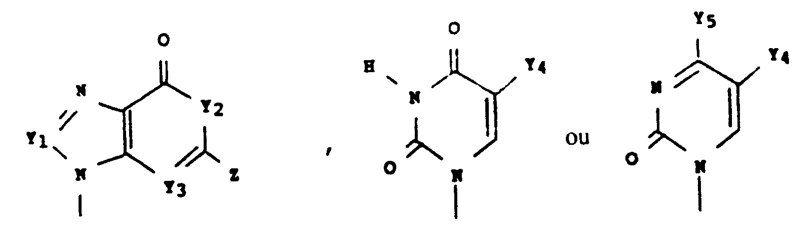 dans laquelle Y₁ est un atome d'azote, un groupe CH, un groupe CCl, un groupe CBr ou un groupe CNH₂ ; Y₂ et Y₃ désignent chacun indépendamment un atome d'azote ou un groupe CH ; Y₄ est l'hydrogène, un groupe C₁-C₄ alkyle, C₁-C₄ alcoxy ou un atome d'halogène ; Y₅ est un groupe amino ou un groupe C₁-C₄ alcoxy ; et Z est l'hydrogène, un atome d'halogène ou un groupe NH₂ ; ou d'un sel pharmaceutiquement acceptable de ce composé, comprenant les étapes suivantes
a) réaction d'un dérivé de 3,5-O-tétraisopropyldisiloxane-1,3-diyl-2-céto-nucléoside avec un arylsulfonylhalométhylidène phosphore ylide pour donner un dérivé de 3,5-O-tétraisopropyldisiloxane-1,3-diyl-2-arylsulfonylhalométhylidène-nucléoside,
b) réaction du dérivé de 3,5-O-tétraisopropyldisiloxane-1,3-diyl-2-arylsulfonylhalométhylidène-nucléoside avec un anion fluorure ou un acide pour donner le dérivé correspondant de 2-arylsulfonylhalométhylidène-nucléoside,
c) réaction du dérivé de 2-arylsulfonylhalométhylidène-nucléoside avec un amalgame d'aluminium/mercure, un amalgame de sodium/mercure ou d'hydrure de tributylétain/azobisisobutyronitrile suivie d'un traitement à l'acide aqueux, et
d) éventuellement réaction du dérivé de 2-arylsulfonylhalométhylidène-nucléoside avec une base.

3. Un procédé pour fabriquer un composé de formule 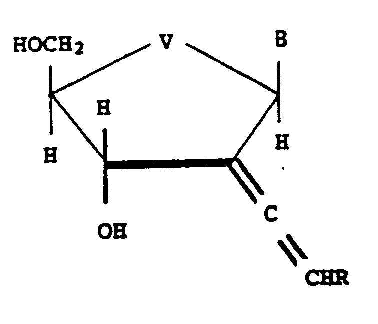 dans laquelle
V est un groupe oxy, méthylène, ou thio,
R est l'hydrogène ou un groupe C₁-C₄ alkyle,
B est un radical de formule 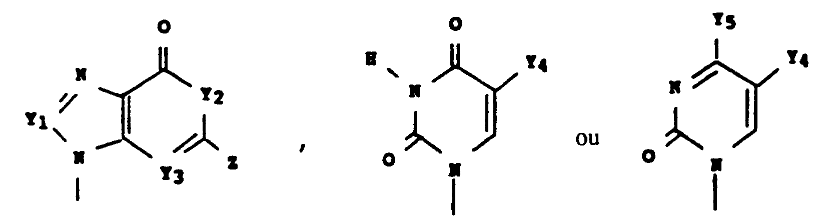 dans laquelle Y₁ est un atome d'azote, un groupe CH, un groupe CCl, un groupe CBr ou un groupe CNH₂ ; Y₂ et Y₃ désignent chacun indépendamment un atome d'azote ou un groupe CH ; Y₄ est l'hydrogène, un groupe C₁-C₄ alkyle, C₁-C₄ alcoxy ou un atome d'halogène ; Y₅ est un groupe amino ou un groupe C₁-C₄ alcoxy ; et Z est l'hydrogène, un atome d'halogène ou un groupe NH₂ ; ou d'un sel pharmaceutiquement acceptable de ce composé, comprenant les étapes suivantes
a) réaction d'un dérivé de 3,5-O-tétraisopropyldisiloxane-1,3-diyl-2-cétonucléoside avec un réactif de Grignard acétylénique pour donner un dérivé de 3,5-O-tétraisopropyldisiloxane-1,3-diyl-2-éthynyl-2-hydroxy-nucléoside,
b) réaction du dérivé de 3,5-O-tétraisopropyldisiloxane-1,3-diyl-2-éthynyl-2-hydroxy-nucléoside avec un agent réducteur tel que l'hydrure de lithium et d'aluminium et le chlorure d'aluminium pour donner le dérivé correspondant de 3,5-O-tétraisopropyldisiloxane-1,3-diyl-2-éthynylidène-nucléoside,
c) éventuellement réaction du dérivé de 3,5-O-tétraisopropyldisiloxane-1,3-diyl-2-éthényldiène-nucléoside avec une base, et
d) réaction du dérivé de 3,5-O-tétraisopropyldisiloxane-1,3-diyl-2-éthénylidène-nucléoside avec un anion fluorure ou un acide.

4. Un procédé pour fabriquer un composé de formule 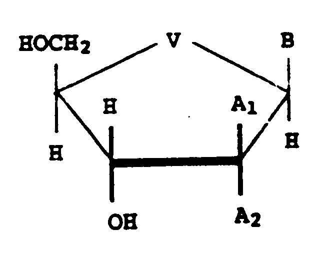 dans laquelle
V est un groupe oxy, méthylène ou thio,
A₁ et A₂ désignent chacun indépendamment l'hydrogène ou un groupe -C≡CR, où R est l'hydrogène ou un groupe C₁-C₄ alkyle à la condition que lorsque A₁ est l'hydrogène A₂ est un groupe -C≡CR, et lorsque A₁ est un groupe -C≡CR, A₂ est l'hydrogène,
B est un radical de formule 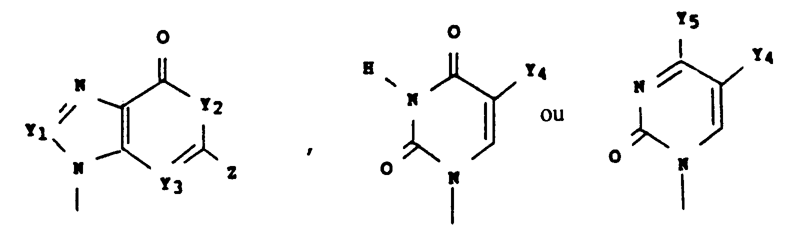 dans laquelle Y₁ est un atome d'azote, un groupe CH, un groupe CCl, un groupe CBr ou un groupe CNH₂ ; Y₂ et Y₃ désignent chacun indépendamment un atome d'azote ou un groupe CH ; Y₄ est l'hydrogène, un groupe C₁-C₄ alkyle, C₁-C₄ alcoxy ou un atome d'halogène ; Y₅ est un groupe amino ou un groupe C₁-C₄ alcoxy ; et Z est l'hydrogène, un atome d'halogène ou un groupe NH₂ ;
ou d'un sel pharmaceutiquement acceptable de ce composé, comprenant les étapes suivantes :
a) réaction d'un dérivé de 3,5-O-tétraisopropyldisiloxane-1,3-diyl-2-céto-nucléoside avec un réactif de Grignart acétylénique pour donner un dérivé de 3,5-O-tétraisopropyldisiloxane-1,3-diyl-2-éthynyl-2-hydroxy-nucléoside,
b) réaction du dérivé de 3,5-O-tétraisopropyldisiloxane-1,3-diyl-2-éthynyl-2-hydroxy-nucléoside avec un agent réducteur tel que le triéthylsilane pour donner le dérivé correspondant de 3,5-O-tétraisopropyldisiloxane-1,3-diyl-2-éthynyl-nucléoside,
c) éventuellement réaction du dérivé de 3,5-O-tétraisopropyldisiloxane-1,3-diyl-2-éthynyl-nucléoside avec une base, et
d) réaction du dérivé de 3,5-O-tétraisopropyldisiloxane-1,3-diyl-2-éthynyl-nucléoside avec un anion fluorure ou avec un acide.

5. Un procédé pour fabriquer un composé de formule 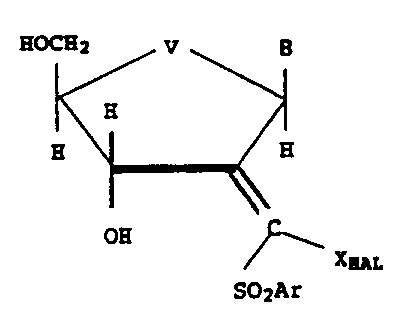 dans laquelle
V est un groupe oxy, méthylène ou thio,
X_{HAL} est un atome d'halogène,
Ar est un groupe C₆-C₁₂ aryle,
B est un radical de formule 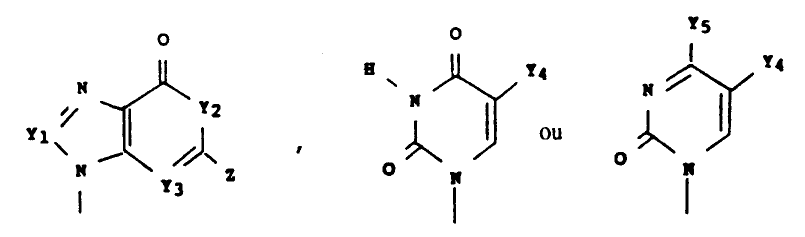 dans laquelle Y₁ est un atome d'azote, un groupe CH, un groupe CCl, un groupe CBr ou un groupe CNH₂ ; Y₂ et Y₃ désignent chacun indépendamment un atome d'azote ou un groupe CH ; Y₄ est l'hydrogène, un groupe C₁-C₄ alkyle, C₁-C₄ alcoxy ou un atome d'halogène ; Y₅ est un groupe amino ou un groupe C₁-C₄ alcoxy ; et Z est l'hydrogène, un atome d'halogène ou un groupe NH₂ ;
ou d'un sel pharmaceutiquement acceptable dudit composé, comprenant les étapes suivantes :
a) réaction d'un dérivé de 3,5-O-tétraisopropyldisiloxane-1,3-diyl-2-céto-nucléoside avec un arylsulfonylhalométhylidène phosphore ylide pour donner un dérivé de 3,5-O-tétraisopropyldisiloxane-1,3-diyl-2-arysulfonylhalométhylidène-nucléoside,
b) réaction du dérivé de 3,5-O-tétraisopropyldisiloxane-1,3-diyl-2-arylsulfonylhalométhylidène-nucléoside avec un anion fluorure ou avec un acide pour donner le dérivé correspondant de 2-arylsulfonylhalométhylidène-nucléoside, et
c) éventuellement réaction du dérivé de 2-arylsulfonylhalométhylidène-nucléoside avec une base.

6. Un procédé selon la revendication 2 pour préparer un composé dans lequel X₁ est un groupe fluoro et X₂ est l'hydrogène.

7. Un procédé selon la revendication 2 pour la préparation d'un composé dans lequel X₁ est l'hydrogène et X₂ est un groupe fluoro.

8. Un procédé selon l'une quelconque des revendications 3 et 4 pour la préparation d'un composé dans lequel R est l'hydrogène.

9. Un procédé selon l'une quelconque des revendications 1 à 4, pour la préparation d'un composé dans lequel V est un groupe oxy.

10. Un procédé selon l'une quelconque des revendications 1 à 4, pour la préparation d'un composé dans lequel Z est l'hydrogène.

11. Un procédé selon l'une quelconque des revendications 1 à 4, pour la préparation d'un composé dans lequel Y₁ est un groupe CH.

12. Un procédé selon l'une quelconque des revendications 1 à 4, pour la préparation d'un composé dans lequel Y₂ est un atome d'azote.

13. Un procédé selon l'une quelconque des revendications 1 à 4, pour la préparation d'un composé dans lequel Y₃ est un atome d'azote.

14. Un procédé selon l'une quelconque des revendications 1 à 4, pour la préparation d'un composé dans lequel Z est un groupe amino.

15. Un procédé selon l'une quelconque des revendications 1 à 4, pour la préparation d'un composé dans lequel Y₂ est un groupe CH.

16. Un procédé selon la revendication 1, pour la préparation d'un composé qui est la 2'-désoxy-2'-difluorométhylidène-cytidine.

17. Un procédé selon la revendication 1, pour la préparation d'un composé qui est la 2'-désoxy-2'-difluorométhylidène-uridine.

18. Un procédé selon la revendication 1, pour la préparation d'un composé qui est la 2'-désoxy-2'-difluorométhylidène-guanosine.

19. Un procédé selon la revendication 2, pour la préparation d'un composé qui est la (Z) ou la (E)-2'-désoxy-2'-fluorométhylidène-cytidine.

20. Un procédé selon la revendication 2, pour la préparation d'un composé qui est la (Z) ou la (E)-2'-désoxy-2'-fluorométhylidène-uridine.

21. Un procédé selon la revendication 2, pour la préparation d'un composé qui est la (Z) ou (E)-2'-désoxy-2-fluorométhylidène-guanosine.

22. Un procédé selon la revendication 3, pour la préparation d'un composé qui est la 2'-désoxy-2'-éthénylidène-cytidine.

23. Un procédé selon la revendication 3, pour la préparation d'un composé qui est la 2'-désoxy-2'-éthénylidène-uridine.

24. Un procédé selon la revendication 3, pour la préparation d'un composé qui est la 2'-désoxy-2'-éthénylidène-guanosine.

25. Un procédé selon la revendication 4, pour la préparation d'un composé qui est la 2'-désoxy-2'(R ou S)-éthynyl-cytidine.

26. Un procédé selon la revendication 4, pour la préparation d'un composé qui est la 2'-désoxy-2'(R ou S)-éthynyl-uridine.

27. Un procédé selon la revendication 4, pour la préparation d'un composé qui est la 2'-désoxy-2'(R ou S)-éthynyl-guanosine.

28. Un procédé selon la revendication 5, pour la préparation d'un composé dans lequel Ar est le groupe phényle.

29. Un procédé selon la revendication 5, pour la préparation d'un composé dans lequel Ar est le groupe phényle et X_{HAL} est du fluor.
